# EUROPEAN PATENT APPLICATION

(11) **EP 1 806 090 A1**
(43) Date of publication of application: **11.07.2007**
(21) Application number: 05790463.3
(22) Date of filing: 04.10.2005
(51) Int. Cl.: A61B 1/00, A61B 10/00, A61B 17/34

(54) **ENDOSCOPE SYSTEM, BIO-SPECIMEN STORAGE CONTAINER, BIO-SPECIMEN SAMPLING METHOD, AND BIO-SPECIMEN TREATING METHOD**

(30) Priority: 05.10.2004 JP 2004292752
(71) Applicant: Olympus Corporation, Tokyo 151-0072 (JP)
(72) Inventor: ITOH, Tomoko Olympus IP Services Co., Ltd., Tokyo 192-8512 (JP); FUKUOKA, Morinao Olympus IP Services Co., Ltd., Tokyo 192-8512 (JP); KAWAI, Tsuyoshi Olympus IP Services Co., Ltd., Tokyo 192-8512 (JP)
(74) Representative: von Hellfeld, Axel
(86) International application number: PCT/JP2005/018379
(87) International publication number: WO 2006/038634

(57) **Abstract**

A sample container (24) containing a biopsy sample obtained by an instrument (15) is arranged in the operation section (4) of an endoscope (2). The sampling means (17) of an instrument (15) inserted into the subject through the instrument channel (20) of the endoscope (2) is used, obtaining a biopsy sample. The biopsy sample is introduced from the instrument (15) into the sample container (24) arranged in the operation section (4), without pulling the instrument (15) outside. The sample obtained can therefore be stabilized fast and be preserved fresh. The biopsy sample is prevented from being contaminated and from contaminating the environment. The sample can therefore undergo accurate examinations and diagnoses.

## Description

### Technical Field

The present invention relates to an endoscope system that can hold a biopsy sample obtained with a medical instrument inserted in the channel of the endoscope, to a biopsy-sample container, a method of obtaining biopsy samples, and a method of treatment biopsy samples.

### Background Art

Generally, biopsy samples are obtained and subjected to pathological examination, biochemical analysis, genomic analysis, or the like. Living samples are obtained with such an instrument as disclosed in, for example, Jpn. Pat. Appln. KOKAI Publication No. 2001-275947 (Patent Document 1). As disclosed in this publication, a puncture needle for use with endoscopes, is inserted into a body cavity through the instrument-insertion channel of an endoscope. The target biopsy tissue is pierced with the puncture needle, while being observing the tissue through the endoscope. The tissue thus pierced is drawn and sampled. The biopsy tissue thus sampled is transported from the endoscopic examination room to another clinical examination room. In the other clinical examination room, the tissue undergoes pathological examination, biochemical analysis, genomic analysis, or the like.

Japanese Patent No. 3517247 (Patent Document 2) and PCT National Publication No. 2001-508674 (Patent Document 3) disclose biopsy-forceps instruments, each attach external for use with endoscopes. Each biopsy-forceps instrument has an instrument part and a ring-shaped collar, both arranged at the distal end of the insertion section of an endoscope, which is inserted into the patient. The instrument part is designed to cut a sample from the biopsy tissue. The collar couples the instrument to the endoscope. The distal end of the insertion section of the endoscope is inserted into the collar, whereby the biopsy-forceps instrument is secured to the endoscope, located adjacent to the insertion section thereof.

The biopsy-forceps instrument has a handle, a sample chamber, a sample-holding member, and a sample sample-holding unit. The handle is provided at the proximal end of the insertion section. When manipulated, the handle operates the instrument part. The instrument part cuts a sample from the biopsy tissue at the distal end of the insertion section. The sample is held in the sample chamber located near the handle.

### Disclosure of Invention

In the case of the instrument disclosed in Patent Document 1, the biopsy sample obtained with the puncture needle for use with endoscopes is transported from the endoscopic examination room to another examination room. In the other examination room, the tissue undergoes various examinations including a pathological examination. With this instrument, it takes long time to perform the next treatment after a biopsy sample is obtained. Particularly, much time is required before the nucleic acid or protein is extracted from the sample or before the sample is fixed. Inevitably, the biopsy tissue sampled may deteriorate. Once the sample has deteriorated, it is difficult to make an accurate diagnosis. Further, to treat the biopsy sample with a reagent, the sample must be transported into a container. While being so transported, the biopsy sample may usually be contaminated by external environment or may contaminate the external environment.

To extract the nucleic acid from a biopsy sample, the sample must be immediately immersed in liquid nitrogen so that the nucleic acid may be stabilized. Handling liquid nitrogen is always accompanied with danger. Further, since the sample is obtained during an operation, the time, manpower and space available for treatment the sampled tissue are much limited. It is therefore desired, in practice, that the biopsy sample and the nucleic acid in the sample be easily and quickly stabilized.

The biopsy samples are obtained during operations. Therefore, the conditions in which biopsy samples are obtained differ from day to day, and the facility with which samples are obtained differs from hospital to hospital. This problem may increase in prominence in the future. Since the condition of stabilizing samples differs, from sample to sample, samples are not standardized. Hence, the results of their analysis can hardly be applied to make diagnoses. Further, means for transporting samples to another room or an outside examination institute is required, in the form of such compact packages that the samples may not be contaminated or contaminate the environment.

Moreover, a system has been desired, which can easily manage a number of samples in the pathological examination room to prevent any medical-staff member to take a sample for another.

With the biopsy- forceps instrument disclosed in Patent Document 2 it is indeed easy to take a biopsy sample from the patient. However, the insertion section to be inserted into the patient has a large diameter, inevitably because the biopsy- forceps instrument is externally attached to the endoscope. This increases the pain and toil on the part of the patient. Further, any sample obtained with the instrument disclosed in Patent Document 2 is exposed to the environment, and cannot be prevented from contaminating or being contaminated. With this instrument it is impossible to obtain liquid samples such as body fluid, secretions or the like, or samples containing liquid and cells. Furthermore, the biopsy samples cannot be fast treated with various treat liquids.

The present invention has been made in view of the foregoing. An object of the invention is to provide an endoscope system, a method of obtaining biopsy samples and a method of treatment biopsy samples, which can treat biopsy samples immediately, thus stabilizing and fixing them and preserving them fresh, can prevent the samples from contaminating the environment or being contaminated, can achieve accurate examination and diagnosis, and can prevent any medical-staff member from taking a sample for another, and can help to standardize the samples among them.

An endoscope system in one aspect of the present invention comprises, an endoscope having, an insertion section which is to be inserted into a subject, an operation section which is coupled to a proximal end of the insertion section and arranged outside the subject, an instrument channel which is arranged in the insertion section and extends from the operation section to a distal end of the insertion section, and observation means for observing an interior of the subject, and an instrument having, an instrument insertion unit which is to be inserted into the subject through the instrument channel, sample-obtaining means arranged at the distal end of the instrument insertion unit and configured to obtain a biopsy sample, and receptacle means arranged at the operation section and configured to contain the biopsy sample obtained by the instrument.

Preferably, the operation section has passage-switching means provided in middle part of the instrument channel, for switching a communication state between a first communication state in which an input port of the instrument channel communicates with an output port of the instrument channel and a second communication state in which the inlet port of the instrument channel communicate with the receptacle means.

Preferably, the receptacle means has at least one container configured to contain a biopsy sample and is able to be removed from the operation section and to be replaced by another.

Preferably, the receptacle means has a filter configured to separate a biopsy sample into a solid sample and a liquid sample.

Preferably, the receptacle means comprises identification means for identifying a biopsy sample to be introduced into the receptacle means.

Preferably, the receptacle means comprises a receptacle device configured to hold a biopsy sample and having, a container which is configured to contain a biopsy sample, and a receptacle unit which is removably attached to the endoscope and configured to hold the container.

Preferably, the receptacle means has a receptacle device which is configured to hold the biopsy sample obtained and which contains a reagent for treatment the biopsy sample.

Preferably, the reagent is an agent for treating biopsy samples.

Preferably, the reagent is an agent for treating nucleic acid, protein, cells, tissues or blood.

Preferably, the reagent is contained in gel.

Preferably, the receptacle means comprises stirring means for stirring the reagent and the biopsy sample in the container.

Preferably, the receptacle means comprises temperature-adjusting means.

Preferably, the biopsy sample obtained by the sample-obtaining means is a biopsy tissue, cells, body fluid, blood or secretion.

A biopsy-sample container for use in an endoscope system comprising, an endoscope having, an insertion section which is to be inserted into a subject, an operation section which is coupled to a proximal end of the insertion section and arranged outside the subject, an instrument channel which is arranged in the insertion section and extends from the operation section to a distal end of the insertion section, and observation means for observing an interior of the subject, and an instrument having, an instrument insertion unit which is to be inserted into the subject through the instrument channel, and sample-obtaining means arranged at the distal end of the instrument insertion unit and configured to obtain a biopsy sample, and receptacle means arranged at the operation section and configured to contain the biopsy sample obtained by the instrument, said container having, a container body, and a coupling part which is configured to be attached and detached to and from the endoscope system.

Preferably, the container body comprises identification means for identifying the biopsy sample.

Preferably, the container body has a filter configured to separate a biopsy sample into a solid sample and a liquid sample.

Preferably, the container body is depressurized.

Preferably, the container body contains a reagent for treatment the biopsy sample.

Preferably, the reagent is stabilizer for nucleic acid.

Preferably, the reagent is an agent for treatment nucleic acid, protein, cells, tissues or blood.

Preferably, the reagent is contained in gel.

A method of obtaining a biopsy sample, in another aspect of the present invention comprises, a insertion step of inserting an instrument insertion unit of an instrument for use with endoscopes, into a subject through an instrument channel of an endoscope configured for observation of the interior of the subject, a biopsy-sample obtaining step of obtaining a biopsy sample by using sample-obtaining means arranged at a distal end part of the instrument insertion unit, and a sample introducing step of introducing the biopsy sample obtained by the sample-obtaining means arranged at the distal end part of the instrument insertion unit, into receptacle means attached to the distal end of the instrument insertion unit.

Preferably, a sample-treatment step of treatment the biopsy sample by introducing the sample into the receptacle means which contains a reagent.

Preferably, a stirring step of stirring the biopsy sample and the reagent in the receptacle means.

Preferably, a culturing step of culturing cells contained in the biopsy sample introduced into the receptacle means.

A method of treatment a biopsy sample, in another aspect of the present invention comprises, a insertion step of inserting an instrument insertion unit of an instrument for use with endoscopes, into a subject through an instrument channel of an endoscope configured for observation of the interior of the subject, a biopsy-sample obtaining step of obtaining a biopsy sample by using sample-obtaining means arranged at a distal end part of the instrument insertion unit, and a sample introducing step of introducing the biopsy sample obtained by the sample-obtaining means, into receptacle means attached to the endoscope, and a treatment step of performing, on the biopsy sample contained in the receptacle means, at least one treat selected from the group consisting of reagent treatment, refrigeration, freezing, freeze-drying, incubation and culture.

Thus, the present invention can provide an endoscope system, a method of obtaining biopsy samples and a method of treatment biopsy samples, which can treat biopsy samples immediately, thus stabilizing and fixing them and preserving them fresh, can prevent the samples from contaminating the environment or being contaminated, to achieve accurate examination and diagnosis, and can prevent any medical-staff member from taking a sample for another, and can help to standardize the samples among them.

### Brief Description of Drawings

FIG. 1A is a side view showing the schematic configuration of an endoscope system according to a first embodiment of the present invention;
FIG. 1B is a plan view showing the distal-end part of an endoscope;
FIG. 2 is a perspective view of the sample sample-holding unit for holding a biopsy sample, which is used in the endoscope system according to the first embodiment;
FIG. 3 is a plan view of the sample container unit provided in the endoscope system according to the first embodiment;
FIG. 4A is a perspective, sectional view of the passage-switching mechanism used in the endoscope system according to the first embodiment, showing the guide path to the container in its closed state;
FIG. 4B is another perspective, sectional view of the passage-switching mechanism, showing an instrument inserted in the guide path to the container;
FIG. 5 is a side view of the sample-holding unit of the endoscope system according to the first embodiment, showing the seal cover in its closed state;
FIG. 6 is a side view of the sample-holding unit of the endoscope system according to the first embodiment, showing the seal cover in its opened state;
FIG. 7A is a schematic diagram explaining how the puncture needle of the instrument is manipulated when the endoscope system according to the first embodiment is operated to obtain a sample;
FIG. 7B is a schematic diagram explaining how the distal-end part of the instrument is pulled toward the insertion port after a sample has been obtained;
FIG. 7C is a schematic diagram explaining how a sampled is drawn into the sample container from the instrument;
FIG. 8 is a side view schematically showing the major components of an endoscope system according to a second embodiment of the present invention;
FIG. 9 is a longitudinal sectional view of the major components of the endoscope system according to the second embodiment, showing the passage-switching mechanism;
FIG. 10A is a longitudinal sectional view of the sample container of the endoscope system according to the second embodiment, showing a modified opening/closing valve in its closed state;
FIG. 10B is a longitudinal sectional view of the major components, showing the opening/closing valve in its opened state;
FIG. 11 is a transverse sectional view of the operation section of the endoscope incorporated in an endoscope system according to a third embodiment of this invention, to which the sample-holding unit has yet to be attached;
FIG. 12A is a transverse sectional view of the operation section of the endoscope incorporated in an endoscope system according to the third embodiment of this invention, showing a sample-holding unit attached to the operation section;
FIG. 12B is a perspective, sectional view of that part of the operation section of the endoscope, to which the sample-holding unit is to be attached;
FIG. 13A is a transverse sectional view showing the guide path to the container, which is closed in an endoscope system according to a fourth embodiment of the present invention;
FIG. 13B is a transverse sectional view an instrument inserted in the guide path to the container;
FIG. 14A is a perspective view showing the sample-holding unit of the endoscope system according to a fifth embodiment of this invention, which has yet to be attached to the operation section of the endoscope;
FIG. 14B is a longitudinal sectional view showing the sample-holding unit attached to the operation section of the endoscope;
FIG. 15 is a perspective view of the sample-holding unit used in an endoscope system according to a sixth embodiment of the present invention;
FIG. 16 is a longitudinal sectional view of the major components of the endoscope system according to the sixth embodiment, showing the sample-holding unit attached to the operation section of the endoscope;
FIG. 17 is a longitudinal sectional view showing the major components of an endoscope system according to a seventh embodiment of this invention;
FIG. 18 is an exploded perspective view showing the major components of an endoscope system according to an eighth embodiment of this invention;
FIG. 19 is a schematic diagram showing the configuration of an endoscope system according to a ninth embodiment of the present invention;
FIG. 20 is a flowchart explaining how the endoscope system according to the ninth embodiment of the invention operates to obtain samples;
FIG. 21 is a flowchart explaining a modified sequence of obtaining samples by using the endoscope system according to the ninth embodiment;
FIG. 22 is a side view of an endoscope system according to a tenth embodiment of the present invention;
FIG. 23A is a schematic diagram illustrating how the containers are transported in the magazine of the endoscope system according to the tenth embodiment, as viewed from the front of the system;
FIG. 23B is a schematic diagram illustrating how the containers are transported in the magazine, as viewed from one side of the system;
FIG. 24 is a longitudinal sectional view explaining how the puncture needle of an instrument is manipulated to obtain a sample in the endoscope system according to an eleventh embodiment of this invention;
FIG. 25 is a longitudinal sectional view explaining how the distal-end part of the instrument is pulled toward the insertion port after a sample has been obtained in the endoscope system according to the eleventh embodiment of the invention;
FIG. 26 is a longitudinal sectional view of the major components of an endoscope system according to a twelfth embodiment of this invention, showing the biopsy-sample obtaining unit removed from a trocar after a sample has been obtained by using the puncture needle of an instrument;
FIG. 27 is a longitudinal sectional view of the major components of the endoscope system according to the twelfth embodiment, explaining how a sample container is attached to the biopsy-sample obtaining unit;
FIG. 28 is a longitudinal sectional view of the major components of the endoscope system according to the twelfth embodiment, explaining how a sample is drawn into the sample container attached to the biopsy-sample obtaining unit of the system; and
FIG. 29 is a perspective view showing the configuration of an endoscope system according to a thirteenth embodiment of the present invention.

### Best Mode for Carrying Out the Invention

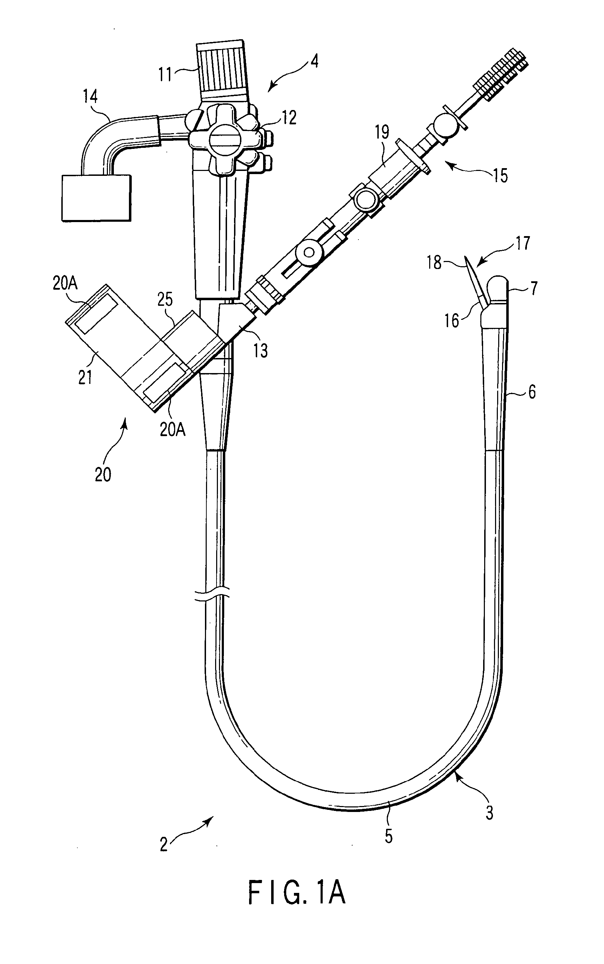

The first embodiment of the present invention will be described with reference to FIGS. 1A to 7C. FIG. 1A is a side view showing the schematic configuration of an endoscope system according to the first embodiment of this invention. In FIG. 1A, reference number 2 designates a soft side-viewing endoscope.

The endoscope 2 has a thin elongated insertion section 3. A broad operation section 4 is coupled to the proximal end of the insertion section 3. The insertion section 3 comprises a thin elongated flexible tube part 5, to bend a bending part 6, and a distal-end part 7. The flexible tube part 5 is coupled at proximal end to the operation section 4. The bending part 6 is coupled at proximal end to the distal end of the flexible tube part 5. The distal-end part 7 is coupled to the distal end of the bending part 6. An operation wire (not shown) is connected to the distal end of the bending part 6.

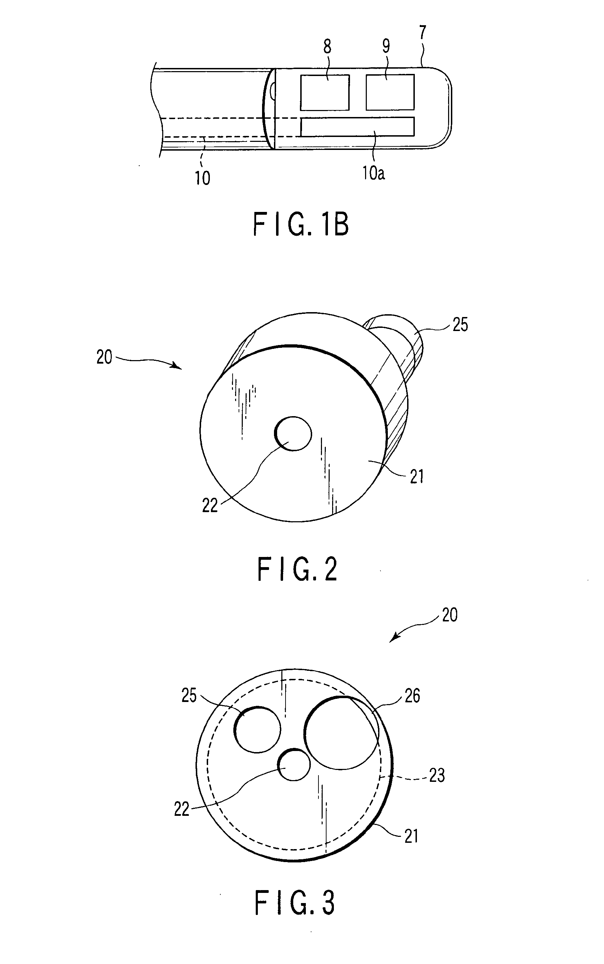

FIG. 1B shows the distal-end part of the insertion section 3. The distal-end part 7 has, on one side, the observation window 8 of an observation optical system, the illumination window 9 of an illumination optical system, and a side hole 10a. The side hole 10a communicates with the distal end of the instrument channel 10 made in the insertion section 3.

At the back of the cover glass of the observation window 8, an objective optical system is arranged. Positioned at the back of the objective optical system is, for example, the distal end of an image-guide fiber. Positioned at the back of the cover glass of the illumination window 9 is the distal end of a light-guide fiber. A forceps-raising base is set in the side hole 10a.

The image-guide fiber, light-guide fiber, instrument channel 10 and operation wire extend toward the operation section 4 through the insertion section 3. The image-guide fiber, light-guide fiber, instrument channel 10 and operation wire are incorporated, as internal components, in the insertion section 3.

An ocular unit 11 is arranged in the operation section 4. The image-guide fiber is coupled, at proximal end, to the ocular unit 11. An endoscopic image is applied to the observation window 8 of the observation optical system and is transmitted to the operation section 4 through the image-guide fiber. The user can therefore observe the endoscopic image at the ocular unit 11. In this embodiment, the endoscope is an optical one in which an endoscopic image is transmitted to the ocular unit 11 through the image-guide fiber. The endoscope may be an electronic endoscope. In this case, an imaging element such as a CCD may convert the endoscopic image may into an electric signal, and the electric signal may be supplied via a signal cable to an external video processor, whereby the endoscopic image is displayed on an external monitor screen.

The operation section 4 further has an operation knob 12 and an instrument-insertion port 13. A universal cord 14 is coupled, at one end, to the operation unit 4. The operation knob 12 is coupled to a bending-operation mechanism that is incorporated in the operation section 4. The operation wire is coupled, at proximal end, to the bending-operation mechanism. When the operation knob 12 is turned, the bending-operation mechanism pulls the operation wire. The bending part 6 is thereby moved by remote control, in accordance with the direction in which the operation knob 12 is turned.

The other end of the universal cord 14 is connected by a connector part to a light-source device. The light-guide fiber extends from the operation section 4, passing through the universal cord 14. Another connector part connects the other end of the light-guide fiber to the light-source device. The light-guide fiber guides the illumination light from the light-source device to the illumination window 9. The illumination light is therefore applied outside from the illumination window 9.

The instrument-insertion port 13 is made in the front part of the operation section 4. The instrument channel 10 is coupled, at proximal end, to the instrument-insertion port 13. Through the instrument-insertion port 13, an instrument 15 for use with endoscopes can be inserted into the instrument channel 10.

The instrument 15 for use with endoscopes has an instrument-insertion part 16 and a sampling means 17. The instrument-insertion part 16 is a thin elongated flexible tube that can be inserted into a body cavity through the instrument channel 10. The sampling means 17 is configured to obtain biopsy samples. In the present embodiment, the sampling means 17 is a tubular puncture needle 18.

A proximal instrument-operation unit 19 is coupled to the proximal end of the instrument-insertion part 16 of the instrument 15. The proximal instrument-operation unit 19 has a device and a needle-operating mechanism. The device has a suction mechanism such as a syringe. The needle-operating mechanism can push and pull the puncture needle 18 from and into the distal end of the tubular part of the instrument-insertion part 16. The syringe of the proximal instrument-operation unit 19 has an outer cylindrical member and a shaft-shaped piston member. The outer cylindrical member has a thin pointed coupling part at the distal end. The coupling part is inserted in the coupling cap of the tube of the instrument-insertion part 16 and is, thus, coupled to the instrument-insertion part 16.

A biopsy-sample obtaining unit (biopsy-sample holding means) 20 is removably coupled to the operation section 4 of the endoscope, in the vicinity of the instrument-insertion port 13. The unit 20 can hold a biopsy sample obtained by using the instrument 15. The biopsy-sample obtaining unit 20 is opposed to the instrument-insertion port 13.

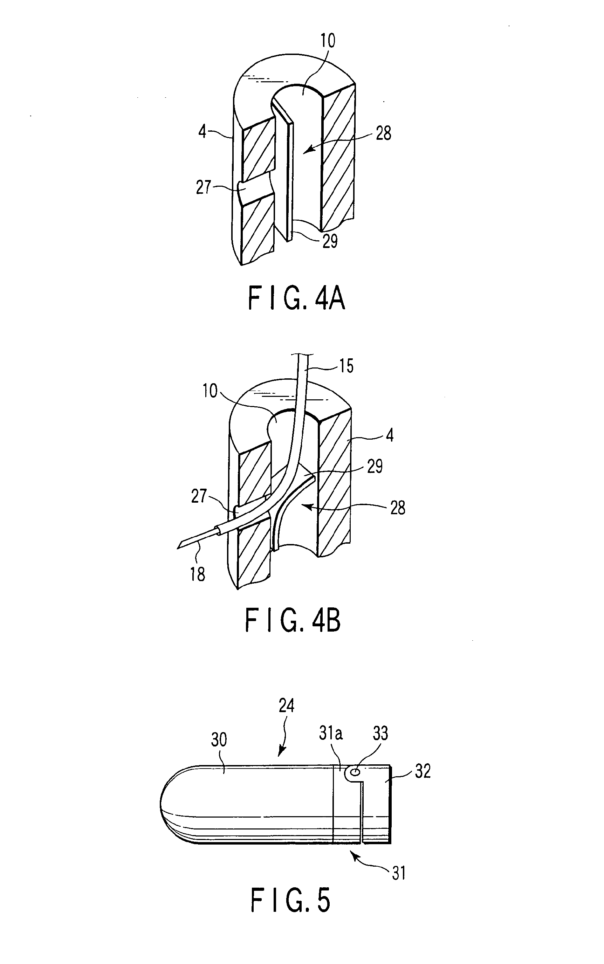

The biopsy-sample obtaining unit 20 has a unit case 21 that is shaped like a hollow cylinder. A shaft 22 is arranged in axial alignment with the unit case 12. As shown in FIG. 3, a rotary member 23 that can rotate around the axis of the shaft 22 is incorporated in the unit case 21. The rotary member 23 has a plurality of container chambers, which are juxtaposed. Sample containers (sample-holding means) 24 are removably inserted in the container chambers. The sample containers 24 will be described later, with reference to FIG. 5. Note that the biopsy-sample obtaining unit 20 has at least one container 24. In this embodiment, a plurality of containers 24 are used and can be rotated around the shaft 22 as the rotary member 23 rotates.

The unit case 21 has, in its one end, an instrument-introducing part 25 and a sample port 26. The instrument-introducing part 25 is shaped like a hollow cylinder. At the sample port 26, a sample can be pulled into, and projected from, the unit case 21. Once the biopsy-sample obtaining unit 20 has been attached to the endoscope 2, the instrument-introducing part 25 is coupled to the operation section 4 of the endoscope 2.

The biopsy-sample obtaining unit 20 may incorporate a temperature-adjusting means 20A. The temperature-adjusting means 20A is constituted by, for example, a resistor or a Peltier element, a temperature sensor, and a temperature-adjusting device. The resistor may be of the type used in ordinary heater, such as nichrome wire, or a sheet heater.

To freeze or cool the sample, the temperature-adjusting means 20A should better have a Peltier element. To insulate the sample thermally, the means 20 should have a Peltier element or a resistor. If frozen or cooled, a biopsy sample obtained is prevented from being degradationd and can be maintained stable.

If a biopsy sample is incuvated, the biopsy tissue sampled or cells sampled can be cultured, treated with enzymes or fixed in appropriate conditions. To be cultured, the tissue or cells should be maintained at 34°C to 38°C, which is close to the patient's internal temperature. The temperature in most intestines is about 38°C. The temperature in some intestines may be 35°C. Cancer cells shrink at temperature of 39°C or more. Hence, cancer cells should not be maintained at 39°C or more. Usually, cancer cells should preferably be maintained at 37 ± 1°C. To process cells or biopsy tissues with enzymes, the temperature is set to one suitable for the process. In order that the cells or tissues be fixed, it is preferable that the temperature be set to one close to room temperature, i.e., approximately 20 to 25°, since they can be fixed at the same condition.

The container 24 can be removed along with the biopsy-sample obtaining unit 20 and can be transported, while the temperature-adjusting means 20A is freezing, cooling or thermally insulating the biopsy sample. Hence, the biopsy sample can be subjected to the next treat, without being influenced.

As shown in FIGS. 4A and 4B, the outer wall of the operation section 4 of the endoscope 2 has a channel-branch hole 27. The sample container 24 can be removably coupled to the channel-branch hole 27. The channel-branch hole 27 communicates with the instrument channel 10. At the junction between the channel-branch hole 27 and the instrument channel 10, a channel-switching mechanism 28 is provided.

The channel-switching mechanism 28 has a channel-switching plate 29. The plate 29 is shaped like, for example, a leaf spring, and opens or closes the inner end of the channel-branch hole 27, thereby to switch the direction in which the instrument 15 for use with endoscopes is guided introduced, while passing through the instrument channel 10. The channel-switching plate 29 is secured to the inner wall of the instrument channel 10, at one end (located at the distal end of the instrument channel 10). The free end of the channel-switching plate 29 can move between a normal position and a sample-guiding position. At the normal position, the plate 29 closes the channel-branch hole 27 as shown in FIG. 4A. At the sample-guiding position, the plate 29 opens the channel-branch hole 27 as shown in FIG. 4B.

As long as the channel-switching plate 29 stays at the normal position as shown in FIG. 4A (while no containers are attached), the channel-switching plate 29 closes the channel-branch hole 27, whereby the channel 10 is sealed airtight. In this state (first communication state), the instrument 15 for use with endoscopes, which passes through the instrument channel 10, is guided toward the side hole 10a made in the distal end of the instrument channel 10).

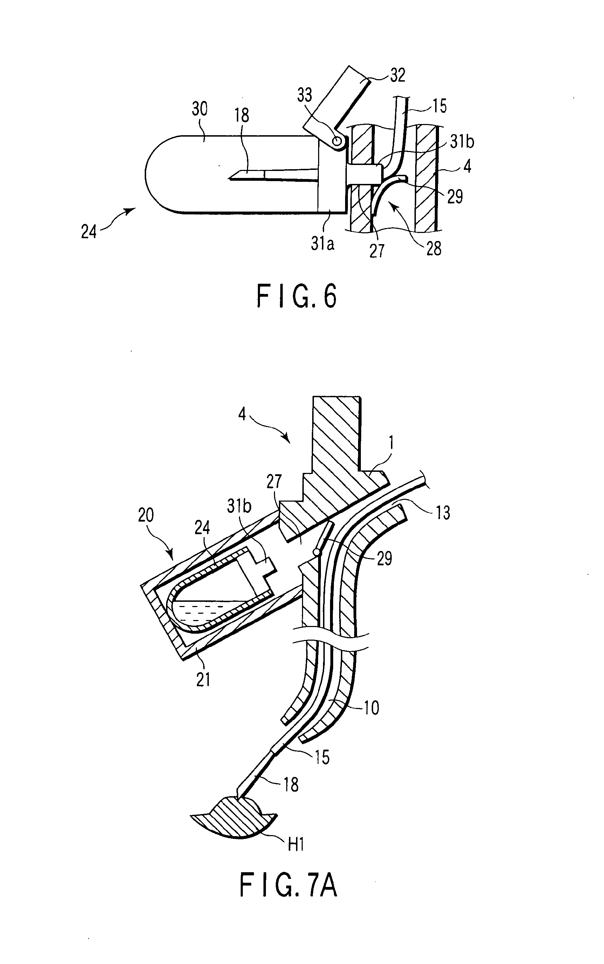

The sample container 24 may be inserted from outside into the channel-branch hole as shown in FIG. 6 (that is, the container is attached). Then, the sample container 24 pushes the channel-switching plate 29, bending the same. As the plate 29 is bent, it is moved to the sample-guiding position as shown in FIG. 4B. In this state, the channel-switching plate 29 closes the passage at the distal end of the instrument channel 10. The instrument 15 for use with endoscopes, which passes through the instrument channel 10, is guided to the channel-branch hole 27 (second communication state).

As shown in FIG. 5, the sample container 24 has a container body 30, an endoscope-coupling part 31, and a sealing cover 32. The container body 30 is a bottomed hollow cylinder. The endoscope-coupling part 31 is attached to the open end the container body 30. The endoscope-coupling part 31 has a cover 31a and a hollow cylindrical part 31b. The hollow cylindrical part 31b has a small diameter and protrudes from the container body 30 in axial alignment therewith. As shown in FIG. 6, the hollow cylindrical part 31b is inserted from outside into the channel-branch hole 27, pushing the channel-switching plate 29 and moving the same to the sample-guiding position.

The sealing cover 32 is rotatably coupled by a hinge part 33 to the outer circumferential surface of the cover 31a. When the sealing cover 32 is rotated around the hinge part 33 with respect to the cover 31a, it can open or closes the opening of the hollow cylindrical part 31b of the cover 31. As shown in FIG. 6, the sealing cover 32 is opened only when the sample container 24 is attached to the operation section 4 of the endoscope 2. When the container 24 is detached from the operation section 4 of the endoscope 2, the cover 32 is closed as shown in FIG. 5, sealing the video-system center 143 again.

The sample container 24 contains a liquid reagent for treatment a biopsy sample, such as RNA-Later. The container 24 is transparent or translucent, either as a whole or in part. Hence, the user can recognize whether the container contains the sample and/or the liquid reagent. The interior of the container 24 is maintained at a pressure lower than the atmospheric pressure. The sample can therefore be automatically drawn into the container 24 from the puncture needle 18 of the instrument 15.

The how the endoscope system 1 so configuration as described above operates will be explained. To obtain a biopsy sample by using the endoscope system 1 according to this embodiment, the biopsy-sample obtaining unit 20 is coupled to the operation section 4 of the endoscope 2. At this time, the biopsy-sample obtaining unit 20 is set, with the endoscope-coupling part 31 aligned with the channel-branch hole 27 made in the outer wall of the operation section 4. Note that a plurality of sample containers 24 have been set beforehand, in the container chambers of the sample obtaining unit 20.

At the start of obtaining biopsy samples, a sample container 24 is held at a waiting position, not inserted yet into the channel-branch hole 27 made in the outer wall of the operation section 4. In this state, the channel-switching plate 29 of the channel-switching mechanism 28 is held at the normal position, where it closes the channel-branch hole 27 as shown in FIG. 4A.

Then, the instrument 15 for use with endoscopes is inserted into the instrument channel 10 through the instrument-insertion port 13. At this time, the channel-switching plate 29 of the channel-switching mechanism 28 closes the channel-branch hole 27 as shown in FIG. 7A. The instrument 15 for use with endoscopes, which is now inserted in the instrument channel 10, is therefore guided to the side hole 10a made in the distal end of the instrument channel 10.

The instrument-insertion part 16 of the instrument 15 for use with endoscopes, thus guide to the distal end through the instrument channel 10, projects from the side hole 10a into the patient. At this point, the forceps-raising base adjusts the direction in which the instrument-insertion part 16 is projecting. That is, the instrument-insertion part 16 is remote-controlled to project in a desired direction. Thus, the instrument-insertion part 16 of the instrument 15 for use with endoscopes has its distal end guided to the site where the target part found in an endoscope diagnosis.

Thereafter, the puncture needle 18 is projected from the distal end of the tube of the instrument-insertion part 16. The puncture needle 18 is thereby thrust into the target part to be examined. At this point, the biopsy tissue H1 (biopsy sample) is sampled from the target part and drawn into the puncture needle 18. Note that the biopsy sample thus obtained includes blood and mucus.

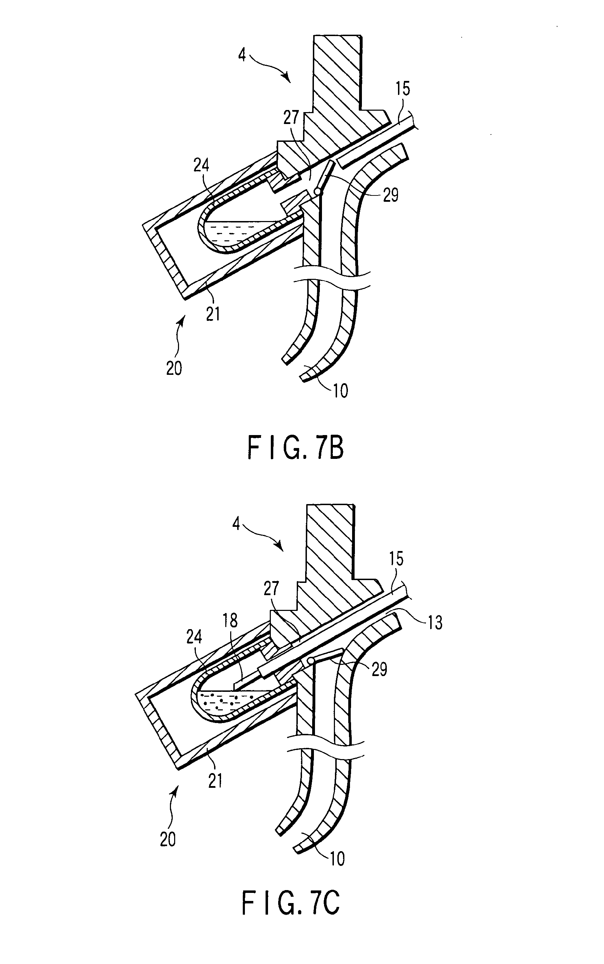

After the sampling has been performed, the puncture needle 18 is drawn into the tube of the instrument-insertion part 16. In this state, the instrument 15 is pulled in such a direction that it may be pulled from the instrument channel 10. At this time, the instrument-insertion part 16 of the instrument 15 is pulled to the position where it has yet to be drawn outside through the instrument-insertion port 13, that is, to a position halfway between the channel-branch hole 27 and the instrument-insertion port 13, as is illustrated in FIG. 7B.

In this state, one sample container 24 is attached to the operation section 4. That is, the empty container 24 is rotated until it faces the channel-branch hole 27. Now that the container 24 lies in this position, a biopsy sample can be transferred from the instrument 15 into the empty container 24.

To attach the sample container 24 to the operation section 4 of the endoscope 2, the cover 32 of the sample container 24 is opened as shown in FIG. 6. The hollow cylindrical part 31b of the sample container 24 is exposed outside. Then, the hollow cylindrical part 31b of the sample container 24 is inserted into the channel-branch hole 27. As the hollow cylindrical part 31b of the sample container 24 is inserted, it pushes the channel-switching plate 29 that lies in the channel-branch hole 27. The channel-switching plate 29 is switched to the sample-guiding position shown in FIG. 4B.

In this state, the instrument 15 for use with endoscopes is pushed into the instrument channel 10 again. The instrument 15 for use with endoscopes, which is pushed again into instrument channel 10, is guided to the channel-branch hole 27 of the instrument channel 10, because the channel-switching plate 29 of the channel-switching mechanism 28 has been moved, closing the channel-branch hole 27 as shown in FIG. 7C. The instrument 15 is therefore inserted into the hollow cylindrical part 31b of the sample obtaining unit 20.

Next, the biopsy sample is pushed out of the instrument 15 and introduced into the sample container 24. This done, the instrument 15 is pulled from the sample obtaining unit 20. The instrument 15 for use with endoscopes is pulled outside from the passes through the instrument-insertion port 13 of the instrument channel 10, after passing through the channel-branch hole 27.

Then, the sample container 24 now containing the biopsy sample is removed from the operation section 4 of the endoscope 2. While the container 24 is being so removed, it remains sealed with the sealing cover 32. The sample container 24 containing the biopsy sample is thus removed through the sample port 26 of the sample obtaining unit 20. To obtain the next sample, another sample container 24 provided in the sample obtaining unit 20 is set in the endoscope 2.

Samples may be acquired from several sites in the patient. If this is the case, the instrument 15 is inserted into the instrument channel 10 of the endoscope 2. The above-mentioned sequence of operations may be repeated, whereby samples can be obtained from various sites in the patient.

The system of the configuration described above attains the following advantages. That is, in the endoscope system 1 according to this embodiment, the sample obtaining unit 20 is attached to the operation section 4 of the endoscope 2. The operation section 4 has the channel-switching mechanism 28 provided in the middle part of the instrument channel 10. The mechanism 28 switches the first communication state to the second communication state, or vice versa. In the first communication state, the inlet port and outlet port of the instrument channel 10 communicate with each other. In the second communication state, the inlet port of the instrument channel 10 communicates with the channel-branch hole 27. Thus, the biopsy sample obtained can be transferred from the puncture needle 18 of the instrument 15 into the sample container 24 provided in the sample obtaining unit 20, without necessity of removing the instrument 25 outside the channel 10 of the endoscope 2, after the instrument-insertion part 16 of the instrument 15 has been inserted into the patient through the instrument channel 10 of the endoscope 2. Hence, the instrument 15 can introduce the sample from the puncture needle 18 of the instrument 15 while it remains inserted into the sample container 24 and sealed from the atmosphere. Therefore, the biopsy sample can prevented from being contaminated or from contaminating the environment. The biopsy sample obtained by the instrument 15 for use with endoscopes can therefore be stabilized faster than otherwise. This helps to accomplish accurate examinations or diagnoses.

Moreover, a biopsy sample, such as tissue, cells or body fluid, can be introduced into the container 24, without being exposed to the environment at all, it has no risk of contamination and is little deteriorated. Hence, biopsy samples are obtained from different subjects under the same condition. The samples are of the same quality, not affected by the medical-staff members who obtained them or by the medical institutes where they are obtained.

In the present embodiment, the sealing cover 32 provided for the sample container 24 is opened only when the sample container 24 is attached to the operation section 4 of the endoscope 2. When the sample container 24 is detached from the operation section 4, the sealing cover 32 is closed again. This prevents air from flowing into the sample container 24 while the sample container 24 remains not attached to the operation section 4 of the endoscope 2. Thus, the sample container 24 is maintained clean, preventing contamination of the interior of the endoscope 2 and the liquid contained in the container 24. In addition, the liquid or sample is prevented from leaking from the sample container 24 of the sample obtaining unit 20.

Moreover, the handling of the biopsy sample is very easy, and the system can be employed in various analyses and examinations. Since the system is a closed one, it can limit the environmental contamination to a minimum. Further, the biopsy samples obtained by the system are easy to transport.

The container 24 should be better sterrized and should not contain nuclease,such as deoxyribonuclease(DNase) or/and ribonuclease(RNase). To sterilize the container 24 or to destroy the enzymes, the container 24 may be autoclaved or treated with EOG (ethylene oxide gas), yrays or reagents (e.g., RNase AWAY manufactured by Molecular BioProducts, Inc.). The sample container 24 may made of glass or plastic. Preferably, it may be made of glass or polymethyl pentane, which is greatly resistant to reagents. If the biopsy sample must be frozen, the container 24 should be made of plastic, polyethylene, polypropylene or the like. If made of plastic, the container hardly adsorb nucleic acid and can be a disposable one. That part of the sample container 24, which serves as a cover (e.g., cover 31a or hollow cylindrical part 31b), should be made preferably of plastic or rubber. If this part is made of plastic or rubber, the container can be sealed after a sample has been introduced into it.

The sample container 24 should be configured to be sealed so that the sample in it may be prevented from contaminating the environment or being contaminated by environment. For example, its cover may be pierced with the puncture needle 18 of the instrument. If so, the container can be easily closed and sealed after the sample obtained has been introduced into it.

To culture cells, it is desired that the cover of the container 24 have a filter that allows passage of oxygen. If the cover of the sample container 24 cannot have an oxygen-passing filter, it is preferably to introduce the sample into the sample container 24, to remove the container 24 from the sample obtaining unit 20 and to seal the container 24 with a cover having an oxygen-passing filer.

In the present embodiment, the container 24 is transparent or translucent, either as a whole or in part. The user can therefore determine whether the container contains the sample and/or the liquid reagent, merely by looking at the container from outside.

Further, the interior of the container 24 should preferable be maintained at a pressure lower than the atmospheric pressure. The sample can therefore be automatically drawn into the distal end of the instrument 15 when the instrument 15 is inserted into the sample container 24. The cover of the container 24, for example, may be pierced with the puncture needle 18 of the instrument 15, which is a sampling means, so that the sample may be transferred into the container. In this case, a pressure difference develops between the container 24 and the puncture needle 18 of the instrument 15. The biopsy sample can therefore be easily drawn into the container in a greater amount than otherwise, even if the sample obtained is of a small amount. This is desirable, particularly when the sample obtained is a liquid.

If the biopsy sample obtained with the sample-obtaining means is a solid, it can hardly be transferred into the container 24 in some cases. In view of this, the container 24 may be depressurized. Then, the sample can be easily transferred into the container 24. The transfer of the sample can more facilitated if the unit 20 holding the instrument 15 and the container 24 has a pushing means that pushes the puncture needle 18 from behind. Preferably, the instrument 15 may have a pushing means having a jig that applies air pressure on the puncture needle 18 or physically pushes the puncture needle 18, from behind.

The unit 20 may hold a container 24 that has a jig that can thrust the puncture needle 18 into the cover of the container 24, can pull the puncture needle 18 from the instrument 15, can apply an air pressure into the unit 20 holding the container 24, from behind, and can physically push the puncture needle 18 from behind. The container 24 may be removed from the unit 20, with the puncture needle 18 thrust in the cover of the container 24, and another pushing means may be used to transfer the biopsy sample into the container 24.

In the embodiment described above, the puncture needle 18 is used as an accessory. Any other accessory can be used in place of the puncture needle 18 if it can introduce the sample into the container. The accessory may be changed to another, in accordance with the sampling site, the amount in which a sample should be obtained and the state of the sample (solid or liquid). The sampling means 17, for example, may not be a puncture needle. Instead, it can be a forceps, a cytology brush, a sampling tube or the like. A sampling tube is a tube, i.e., a hollow member, and usually has an outer diameter of 1 to 10 mm and an inner diameter of 0.5 to 8 mm, though the outer diameter is not limited to this particular value. Preferably, it is made of soft plastic or rubber. If a sampling tube of this type is used, it is inserted into a duct in the patient (e.g., the pancreatic duct or the bile duct). The secretion is sampled from the duct and passed through a filter, which filters out the exfoliated tissue or cells. The tissue or cells thus sampled can be subjected to an examination.

The container 24 to contain a sample obtained may contain, beforehand, reagents for treatment the biopsy sample. The reagents that can be used are selected in accordance with the biopsy sample obtained, the substances (e.g., protein or nucleic acid) contained in the biopsy tissue or body fluid or the kind of the treatment to be carried out.

For example, the nucleic acids contained in the biopsy tissue or body fluid sampled, particularly RNA, may be analyzed. RNA is more readily degradationd by the RNase existing in the environment than any other nucleic acid present in biopsy tissue or body fluid. The RNase exists in, for example, the cells and human sweat. To achieve accurate analyses and acquire correct analysis results, the sample must not be exposed the environment so that the nucleic acids may not undergo decomposition. To analyze nucleic acids, the acids are amplified in most cases. If nucleic acids other than that contained in the sample to be analyzed or substances containing nucleic acids mixes into the biopsy sample, they may be amplified, too, in some cases.

It is therefore important to shorten the time that lapses after the biopsy sample is obtained until the nucleic acid is stabilized. To stabilize the nucleic acid, it is desirable to use reagents that inhibit the activity of the enzyme that degradations the nucleic acid. Nuclease inhibitor, ribonuclease inhibitor, deoxyribonuclease inhibitor, or surfactant, for example, should better be applied. Among the commercially available reagents are, for example, RNAlater (manufactured by Ambion, Inc.) or ethanol. If RNAlater or ethanol is used, the nucleic acid can remain stabilized for a long time at room temperature. Hence, liquid nitrogen or thermal insulator, which may impose some risk, or an apparatuses such as a refrigerator or a freezer need not be used. The sample can be transported with ease and in safety. The use of RNAlater or ethanol is therefore desirable. (Hitherto, an accessory, such as a puncture needle, is removed from the main unit of the endoscope, then collected outside the endoscope, and frozen in liquid nitrogen, and stored.)

The sample container 24 may contain reagents so that the cells or tissues may be fixed or preserved after they are taken into the container 24. The reagents that may be contained are, for example, a fixation solution, a preservation solution/storage solution and a cell-treatment reagent. The fixing and fixation solution may be alcohol (e.g., methanol, ethanol or propanol), formalin and Michael solution. The cell-treatment liquid may be one sold by Cytec, Inc. or Tripath, Inc. If the sample is blood or contains blood and the coagulation of blood may cause a problem, a anticoagulant (e.g., sodium citrate, heparin, potassium salt of EDTA, or the like) can be applied.

The treatment of the biopsy sample includes various treatment, not only stabilization, fixing and preservation, all mentioned above, but also culture, dying, washing, blocking, enzyme treatment and stimulation. Therefore, reagents for culture, dying, washing, blocking, enzyme treatment and stimulation can be contained, as well, in the sample container 24.

Sampled cells and tissues can be dyed and examined under the same condition after they have been preserved or fixed. Hence, they can be examined more accurately than otherwise.

What is most important with pathological examination is to examine cells or tissues within so short a time as possible. Sampled tissues start undergoing decomposition once the supply of blood to them has stopped. Thus, once tissues are sampled, and the supply of blood to them is stopped, they become unable to be examined before long, because they have been degradationd to a large extent even if they have immersed in fixation solution.

Nevertheless, a biopsy sample, such as a tissue or cells, can be subjected to correct examination if it is immersed in a treat liquid (e.g., fixing liquid or fixation solution) immediately after it has been obtained from the patient and is thereby prevented from being degradationd. One of the most generally used fixing and fixation solutions is 10%-neutral buffer formalin solution. To treat a sampled tissue, this formalin solution must be used in an amount at least ten times the amount of the sample. Formalin has toxicity, is considered to be a stimulant to the skin and the mucous membrane, and is suspected as a carcinogen. In the present invention, the sample can undergo various treatment within the sample container. This prevents the sample from contaminating the environment, enhancing the safety for the members of the medical staff.

The present invention can greatly shorten the time that lapses after the biopsy sample is obtained until it is treated. Therefore, the sample can undergo various treatment, such as stabilization, before it is deteriorated.

Further, only if the container 24 contains culture medium, buffer solution or physiological salt solution, it can hold a sampled tissue or sampled cells alive. Various kinds of examinations can be made, or the biopsy tissues can be cultured, with the container 24 removed from the unit 20.

To culture adhesive cells, the inner surface of the container 24 may be coated with polyethyleneimine or collagen. Preferably, the container 24 may preferably have flat surfaces to which cells may adhere.

The sample container 24 may have a stirring means that can stir the reagent and the biopsy sample, both contained in the container 24. In this case, the stirring means stirs the reagent and biopsy sample in the container 24, thus promoting the treat being performed on the biopsy sample.

The biopsy sample obtained may be a biopsy tissue, cells, body fluid, blood and secretion. A biopsy tissue is a collection of cells and is, for example, a cancer lesion or a normal part. A secretion is a substance generated from cells. Objects to sample may be gastric juice, pancreatic juice, bile, lymphatic fluid, peritoneal juice, thoracic juice and lung lavage fluid. The exfoliated tissue, exfoliated cells and nucleic acid existing in a liquid sample may be removed by means of filtering or centrifugal separation, and may then be subjected to various examinations.

The pancreatic juice sampled, for example, may contain cells. To analyze the nucleic acid in these cells, the cells should better be separated from the pancreatic juice and then be subjected to various treatment (e.g., stabilization or extraction of the nucleic acid). The protein or nucleic acid contained in the pancreatic juice can be analyzed.

The biopsy sample container 24 may contain beforehand a treatment solution containing phenol or guadine thiocyanate (for example, ISOGEN (manufactured by Nippon Gene), TRIZOL (manufactured by Inbitrto Gene)). Then, the biopsy sample introduced into the container 24 is stirred in the endoscope (unit), and the biopsy sample container is removed from the endoscope (unit). The sample may then be immediately frozen in a freezer or with liquid nitrogen. The sample may be frozen at -70°C or less. Alternatively, a treat liquid such as chloroform may be added, and various treatment such as centrifugal isolation may then be performed, as is needed in accordance with the objective, thereby to separate RNA, DNA and protein from one another or to extract them independently.

In order to extract RNA, DNA and protein from one sample, the sample container may preferably contain phenol, guanidinium compound having concentration of about 0.5 to 2 M (e.g., acidic guanidium compound or its salt, such as guanidium thiocyanate or guadinium chlorinate), a buffer solution and a phenol-soluble agent. Then, RNA, DNA and protein, not degradationd, can be extracted.

The sample contained in the sample container may be freeze-dried, not refrigerated, frozen or incuvated. If freeze-dried, the sample can be dried by sublimation. Then, it will scarcely change in composition, and a loss of volatile components is small. Therefore, the sample can be stabilized, with little damage, and can be preserved for a long time. In this case, it is desirable to introduce into the sample container a solution having osmotic pressure substantially equal to that of the sample, for example, a buffer solution such as lxPBS or TBS of an appropriate concentration. To analyze the nucleic acid, the solution should contain no nuclease, such as a deoxyribonuclease(DNase) or a ribonuclease(RNase). The solution should better contain reagents that inhibit the activity of nuclease, such as nuclease inhibitor, ribonuclease inhibitor, deoxyribonuclease inhibitor and surfactant. If the solution contains these reagents, it will fast stabilize the nucleic acid.

To perform freeze-drying on the biopsy sample, the sample obtaining unit 20 may have a freeze-drying means. If the unit 20 has a freeze-drying means, however, the system will be large. In view of this, the sample container may be removed and the sample in the container may be freeze-dried by a commercially-available freeze-drying apparatus. A sample may be introduced into a sample container already containing, for example, 1xPBS, and may be dried at a temperature of -20°C or less and a pressure of 100 Pa or less, while being cooled, and then the temperature and the pressure may be increased back to normal values. Thus, the sample can be freeze-dried.

The reagent may be gel-encapsulated and stably held in the sample container 24. This facilitates the handing of the container that contains no biopsy samples. After a biopsy sample has been introduced into the container, the reagent does not act on a part of the biopsy sample only. The reagent therefore uniformly mixes with the sample. The sample is thereby treated uniformly.

It is desired that the endoscope 2 or the sample obtaining unit 20 (i.e., sample-holding device) has a mixing means that mixes such a reagent as specified above with the sample. The mixing means can make the sample and the reagent mix together fast, preventing the sample from being degradationd or deteriorated, and make them contact each other uniformly. A desirable stirring means may be one that has an ultrasonic vibrator or one that can rotate or vibrate the sample-holding unit 20, or may be one that can rotate or vibrate the container 24. Alternatively, the container 24 may incorporate a magnetic body, which is driven with a magnetic force applied externally.

The container may contain thixotropic gel (i.e., gel whose apparent viscosity decreases with time at a constant shear rate and gradually regains the initial value the shear strain is removed from it). If the mixing means for mixing the reagent and the sample is provided, the sample can be separated by virtue of the difference in specific gravity. For example, the sample may be blood and a gel having a specific gravity of about 1.043 to about 1.050 g/cm³ may be used. If this is the case, the plasma of the blood will adsorb to the gel surface, separated from the remaining part of the blood, after the blood has been mixed with the gel.

Thixotropic copolymer gel is insoluble in water and is almost reagently inactive in blood. The gel can be made from dimethyl polysiloxane or from polyester and precipitated methylate silica. The above-mentioned gel used has a specific gravity ranging from about 1.40 to about 1.080 g/cm³.

The container 24 for the biopsy sample obtained may have a filter. The filter can be used for various purposes. The filter may separate a liquid biopsy sample and a solid biopsy sample from each other. Alternatively, the filter may have the function of adsorbing nucleic acid, in order to extract the nucleic acid. Preferably, the container 24 should be depressurized, to enable the filter to perform filtration easily.

The container 24 for containing a biopsy sample should preferably labeled with a barcode or an IC tag. Then, the patient, the site at which the sample has been acquired, the date of sampling, and like can be easily read from the barcode or IC tag.

The endoscope 2 and the container 24 for containing a biopsy sample, or the unit 20 holding the container 24 should better be transparent in part. Then, the user can confirm whether the biopsy sample has been reliably transferred into the container 24.

After the sample has been thus introduced into the container 24 or treated, it be cooled, frozen or freeze-dried. The container 24 is then transferred to an examination room or an examination center, where the samples can appropriately undergo various examinations.

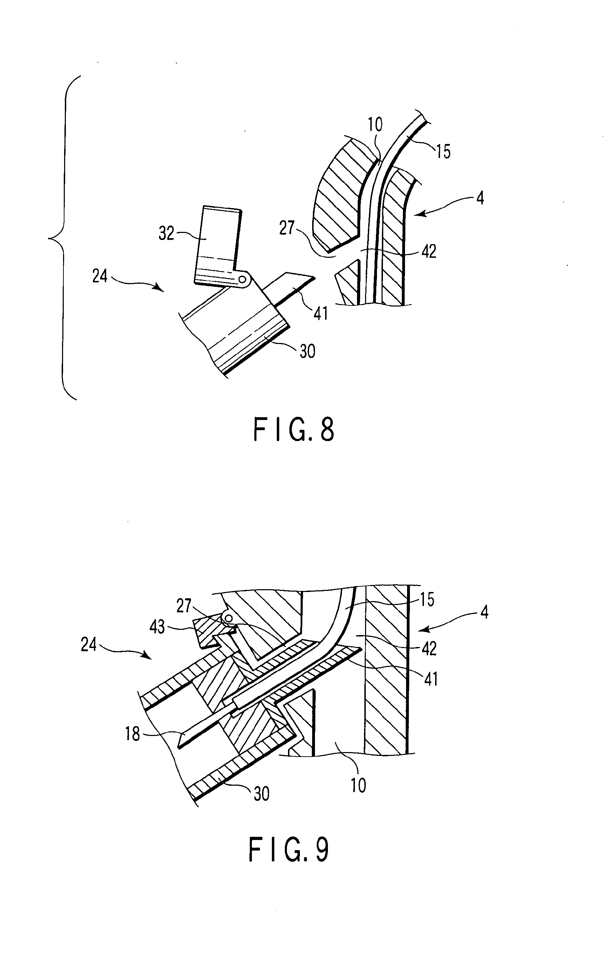

FIGS. 8 and 9 show a second embodiment of the present invention. This embodiment differs from the endoscope system 1 (see FIGS. 1A to 7C) according to the first embodiment in that the channel-switching mechanism 28 is changed in configuration as will be described below. In any other respect, the present embodiment is identical in configuration to the first embodiment. The component identical to those of the first embodiment are designated by the same reference numbers and will not be described.

The present embodiment has no component equivalent to the channel-switching plate 29 used in the first embodiment. Instead, a projection 41 protrudes from the distal end of the hollow cylindrical part 31b of the sample container 24. The projection 41 can be inserted into, and removed from, the interior of the instrument channel 10. The distal end of the projection 41 is cut slantwise with respect to the hollow cylindrical part. The projection 41 guides the instrument 15 for use with endoscopes, which extends through the instrument channel 10, toward the channel-branch hole 27.

FIG. 8 illustrates the sample-holding unit 20 that has yet to be attached to the sample container 24. The channel 10 through which the instrument 15 extends has a space 42 in the middle part. The projection 41 protruding from the hollow cylindrical part 31b of the sample container 24 can enter this space 42. Beside the space 42, the channel-branch hole 27 is arranged, through which the instrument 15 is inserted into the container 24.

As shown in FIG. 9, a hook-shaped stopper member 43 is provided on the outer surface of the operation section 4. The stopper member 43 is designed to hold the sample container 24 at, and release the same from, the operation section 4. To attach the sample container 24 to the operation section 4, the hollow cylindrical part 31b of the sample container 24 is inserted into the channel-branch hole 27 of the instrument channel 10, whereby the stopper member 43 is fitted in the open end of the container body 30 of the sample container 24.

While the sample container 24 of the sample obtaining unit 20 remains not used, the channel-branch hole 27 is covered with a rubber cover (not shown). The instrument channel 10 is thereby maintained in airtight state.

How the second embodiment operates will be explained. In this embodiment, the space 42 made in the instrument channel 10 remains open until the sample container 24 is coupled to the outer surface of the operation section 4. The instrument 15 for use with endoscopes, inserted into the instrument channel 10 through the instrument-insertion port 13 is guided through the instrument channel 10 to the side hole 10a made in the distal end of the instrument channel 10. Thus, the puncture needle 18 at the instrument-insertion part 16 can be thrust into the sampling site in the patient. A part H1 of the biopsy tissue at the sampling site can therefore be introduced into the puncture needle 18. Thus, the biopsy sample H1 is obtained.

After the sampling, the instrument15 is moved in such a direction that it is pulled from the instrument channel 10. At this time, the instrument-insertion part 16 of the instrument 15 is pulled to the position where it comes out of the instrument-insertion port 13, that is, to a position halfway between the channel-branch hole 27 and the instrument-insertion port 13, as is illustrated in FIG. 7B.

In this state, the sample container 24 is attached to the operation section 4. At the time of attaching the container 24 so, the hollow cylindrical part 31b of the sample container 24 is inserted into the channel-branch hole 27 of the operation section 4 in FIG. 9. The projection 41 protruding from the hollow cylindrical part 31b is thereby inserted into the insertion space 42 provided in the channel 10. The projection 41 closes the passage that is closer to the side hole 10a than to the channel-branch hole 27 of the operation section 4. The instrument 15 for use with endoscopes is therefore guided to the channel-branch hole 27 of the operation section 4 when it is pushed again into the instrument channel 10. The instrument 15 is thereby inserted into the hollow cylindrical part 31b of the sample container 24. The biopsy sample is pushed from the instrument 15 and introduced into the sample container 24.

In the above-described configuration, the projection 41 projects form the distal end of the hollow cylindrical part 31b of the sample container 24 and can be inserted into the instrument channel 10 and guides, to the instrument 15 for use with endoscopes, the instrument 15 that passes through the instrument channel 10. Therefore, the channel-switching plate 29 need not be used as in the first embodiment. The channel-switching mechanism 28 can therefore be simplified in configuration.

Moreover, in the present embodiment, the stopper member 43, which is provided at the channel-branch hole 27 of the operation section 4, holds the sample container 24 in position. A sample can therefore be acquired without using the sample obtaining unit 20. Thus, in the case where a plurality of containers 24 need not be used, the sample can be easily obtained, while maintaining the interior of the endoscope 2 and the container 24 in airtight state.

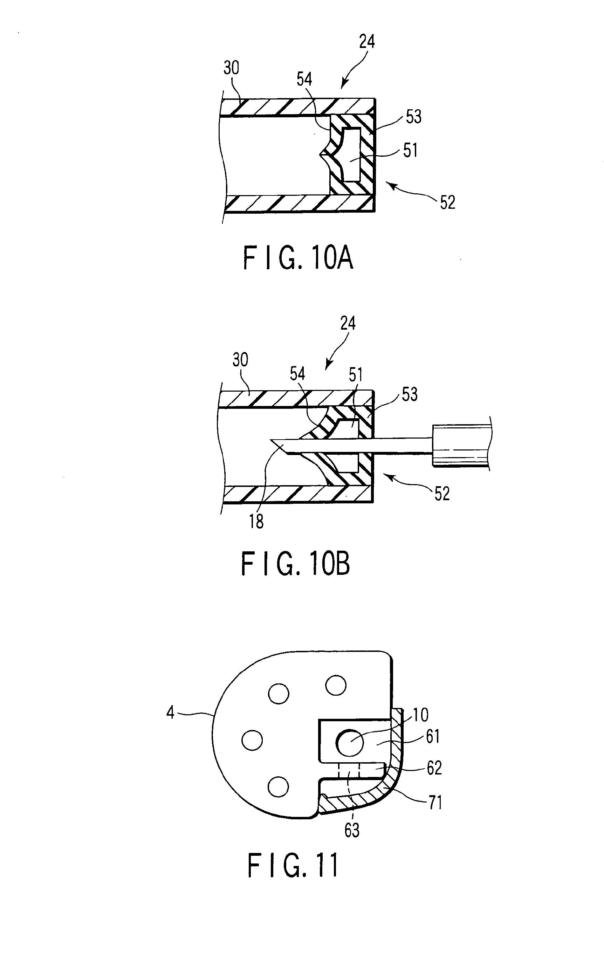

FIGS. 10A and 10B show a modified means for sealing the sample container 24 held in the sample obtaining unit 20 of the second embodiment (see FIGS. 8 and 9). The cover 32 of the sample container 24 is replaced by an opening/closing valve 52 as shown in FIG. 10A. The valve 52 is made of elastic material such as rubber and has an airtight chamber 51.

The opening/closing valve 52 has at least one partition 53 and a backflow check valve 54. The partition 53 has not holes. Before use, the container 24 has its interior partitioned by the partition and the backflow check valve 54 as shown in FIG. 10A. The interior of the body 30 of the container 24 is therefore maintained airtight.

When the instrument 15 for use with endoscopes is guided to the channel-branch hole 27 of the instrument channel 10, the puncture needle 18 attached to the instrument 15 is thrust into the partition 53 and backflow check valve 54 of the opening/closing valve 52. At this point, the puncture needle 18 pierces the partition 53, making a hole in the partition 53. Nonetheless, the interior of the container body 30 remains airtight, because the elasticity of the partition 53 and the use of the backflow check valve 54.

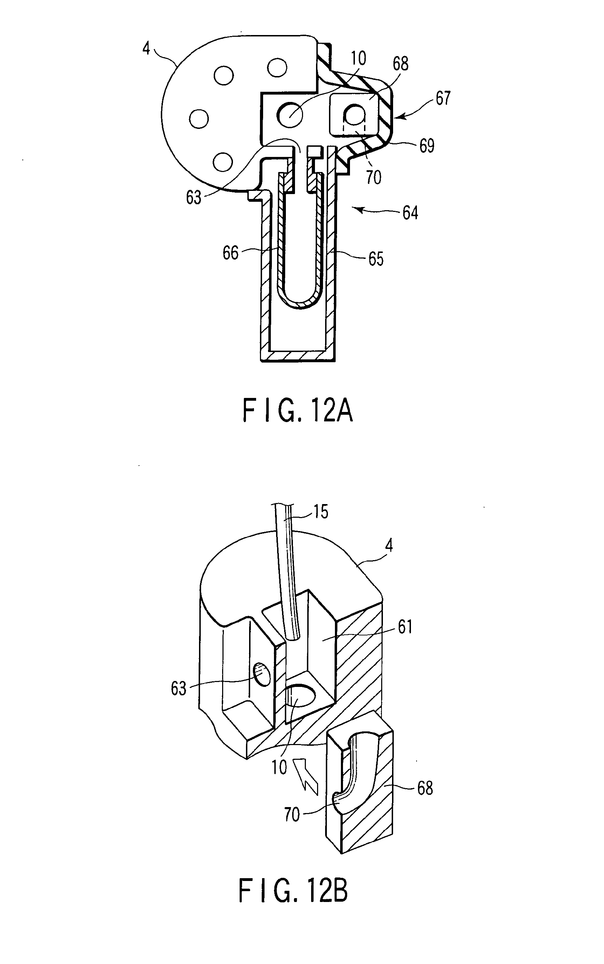

FIGS. 11, 12A and 12B show a third embodiment of the present invention. This embodiment differs from the first embodiment (see FIGS. 1A to 7C) in that the channel-switching mechanism 28 of the endoscope system 1 is changed in configuration as will be described below. In any other respect, this embodiment is identical to the first embodiment. The components identical to those of the first embodiment are designated by the same reference numbers and will not be described.

That is, in this embodiment, the operation section 4 of the endoscope 2 has a block receptacle 61 as shown in FIG. 11. The block receptacle 61 is provided at the middle part of the instrument channel 10. The side wall of the block receptacle 61 has a channel-branch hole 63. This channel-branch hole 63 communicates with the interior of the block receptacle 61.

As shown in FIG. 12A, a biopsy-sample obtaining unit 64 is removably attached to the operation section 4 of the endoscope 2. This biopsy-sample obtaining unit 64 comprises a unit body 65, a sample container 66, and a channel-switching mechanism 67. The channel-switching mechanism 67 comprises a channel-switching block 68 and a rubber member 69. The rubber member 69 holds the channel-switching block 68. The sample container 66 is coupled to the channel-branch hole 63.

The channel-switching block 68 has a communication passage 70, which can connect the upstream end of the instrument channel 10 (i.e., at the instrument-insertion port 13) to the channel-branch hole 63. The rubber member 69 has the function of holding the channel-switching block 68 at a position, outside the block receptacle 61, as shown in FIG. 12A, while the interior of a body cavity is being observed through the endoscope 2 or while a sample is being acquired. When the user pushes the rubber member 69 from outside, the block 68 is set in the block receptacle 61. The block 68 therefore closes the passage located at the downstream end of the instrument channel 10 (i.e., at the side hole 10a provided at the distal end of the instrument channel 10). The communication passage 70 therefore connects the passage at the upstream the instrument channel 10 (i.e., at the instrument-insertion port 13) to the channel-branch hole 63. Thus, the channel-switching mechanism 67 has the function of moving the instrument 15 inserted into the instrument-insertion port 13 through the instrument channel 10, between a normal position and a switched position. At the normal position, the instrument 15 lies at the side hole 10a. At the switched position, the instrument 15 reaches the sample container 66 after passing through the communication passage 70 made in the block 68 and through the channel-branch hole 63.

A rubber cover 71 is secured to the operation section 4. The rubber cover 71 closes the block receptacle 61 and the channel-branch hole 63 as shown in FIG. 11, thus maintaining the instrument channel 10 in airtight state, while the biopsy-sample obtaining unit 64 remains not used.

The configuration described above is advantageous in the following respect. The switching block 68 is moved between the normal position, where it lies at the side hole 10a, and the switched position, it pushes the block 68 into the block receptacle 61, with the biopsy-sample obtaining unit 64 attached to the operation section 4 of the endoscope 2. Therefore, the instrument channel 10 can be easily switched.

Note that the channel-switching mechanism 67 may be incorporated in the main unit of the endoscope 2.

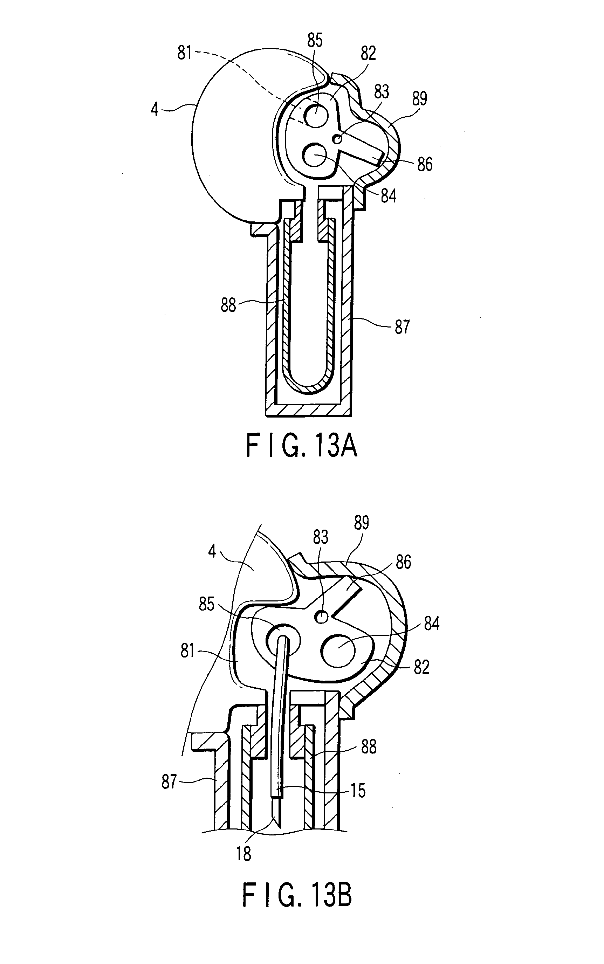

FIGS. 13A and 13B show a fourth embodiment of this invention. The present embodiment differs from the first embodiment (see FIGS. 1A to 7C) in that the channel-switching mechanism 28 of the endoscope system 1 is changed in configuration as will be described below. In any other respect, this embodiment is identical to the first embodiment. The components identical to those of the first embodiment are designated by the same reference numbers and will not be described.

In this embodiment, the operation section 4 of the endoscope 2 has a cylinder receptacle 81 as illustrated in FIG. 13A. In the cylinder receptacle 81, a channel-switching cylinder 82 is mounted on a support shaft 83 to rotate around the shaft 83. The cylinder 82 has two (first and second) passages 84 and 85 and an operation lever 86. The instrument channel 10 is branched to the passages 84 and 85.

The first passage 84 is a straight hole, which guides the instrument 15 to the distal end of the endoscope 2 at the time of observing the sampling site in the patient or acquiring a sample from the site. The second passage 85 connects the instrument-insertion port 13 to the sample container 88 held in a biopsy-sample obtaining unit 87.

A rubber cover 89 is provided outside the cylinder receptacle 81 and the biopsy-sample obtaining unit 87. This rubber cover 89 closes the cylinder receptacle 81, sealing the receptacle 81 from the atmosphere. The instrument channel 10 is thereby maintained in airtight state.

To operate the channel-switching mechanism 28, the user rotates the operation lever 86 of the cylinder 82 outside the rubber cover 89. As the cylinder 82 is so rotated as shown in FIG. 13A, the first passage 84 is aligned with the channel 10. Then, the instrument 15 is guided to the distal end of the endoscope 2.

As the operation lever 86 is rotated, the cylinder 82 rotates as shown in FIG. 13B. When the second passage 85 is aligned with the channel 10, the instrument 15 is guided into the sample container 88.

The configuration described above is advantageous in the following respect. The instrument channel 10 can be switched when the operation lever 86 of the cylinder 82 is rotated outside the rubber cover 89, while the biopsy-sample obtaining unit 87 remains attached to the operation section 4 of the endoscope 2. The instrument channel 10 can therefore be switched with ease.

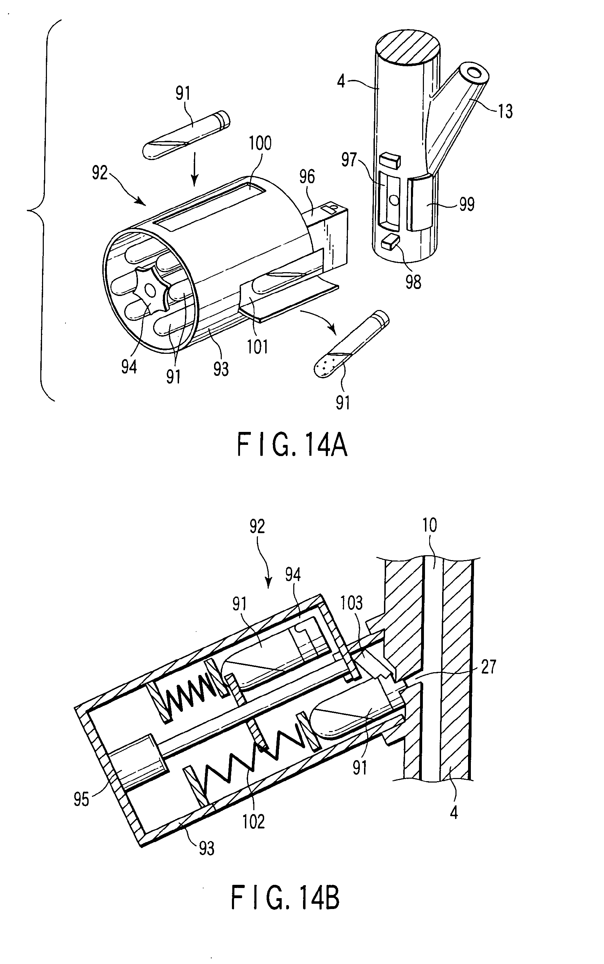

FIGS. 14A and 14B show a fifth embodiment of this invention. The present embodiment differs from the first embodiment (see FIGS. 1A to 7C) in that the sample obtaining unit 20 of the endoscope system 1 is changed in configuration as will be described below. In any other respect, this embodiment is identical to the first embodiment. The components identical to those of the first embodiment are designated by the same reference numbers and will not be described.

As shown in FIG. 14A, this embodiment has a drum-shaped, biopsy-sample obtaining unit 92 that holds a plurality of sample containers 91. The biopsy-sample obtaining unit 92 has a hollow-cylindrical magazine 93.

A cylinder 94 and a drive means 95 are arranged in the magazine 93. The cylinder 94 holds the containers 91 in it. The drive means 95 rotates the cylinder 94. Further, as shown in FIG. 14B, an endoscope-coupling part 96 is provided in the cylinder 94, protruding from the bottom of the magazine 93.

The operation section 4 of the endoscope 2 has a recess 97 that can hold the biopsy-sample obtaining unit 92. The recess 97 has a shape complementary to the endoscope-coupling part 96 of the magazine 93. Thus, the endoscope-coupling part 96 can be fitted in the recess 97.

Further, the operation section 4 has a locking mechanism 98, a button 99, and a lever. The locking mechanism 98 can lock the endoscope-coupling part 96 in the recess 97 and release the same from the recess 97. The button 99 and the lever may be pushed and rotated, respectively, to make the mechanism 98 lock the endoscope-coupling part 96 in the recess 97 of the operation section 4 or releases the part 96 from the recess 97.

FIG. 14A illustrates the biopsy-sample obtaining unit 92 that has yet to be attached to the operation section 4 of the endoscope 2. FIG. 14B depicts the biopsy-sample obtaining unit 92 that has been attached to the operation section 4 of the endoscope 2.

The magazine 93 has an upper opening 100 in the upper part, and an ejection opening 101 in the lower part. Containers 91 not used yet are loaded into the magazine 93 through the upper opening 100 and are arranged in a circle in the cylinder 94 of the magazine 93.

The drive means 95 provided in the cylinder 94 is driven by, for example, a motor. Alternatively, it may be manually driven, by using a lever. The drive means 95 can therefore rotate the cylinder 94. The containers 91 loaded in the cylinder 94 can therefore be moved in a circuit, around the axis of the magazine 93. The containers 91 can be distinguished, one from another, because of the barcodes or the like printed on them. A desired container 91 can therefore be moved to a desired position.

The magazine 93 has spring members 102 that bias the containers 91 toward the endoscope-coupling part 96, respectively. Each container 91 can be pushed by the spring member 102 into the endoscope-coupling part 96 when it comes into alignment with the endoscope-coupling part 96. As the container 91 so pushed, its cover 103 opens, and the container 91 is inserted into the channel-branch hole 27 of the operation section 4 and connected to the operation section 4 of the endoscope 2. At this time, the spring member 102 pushes the container 91 onto the operation section 4. The container 91 is therefore closes the channel-branch hole 27. Hence, anything in the container 91 is not contaminated, and anything outside the container 91 is not contaminated, either.

In this state, the instrument 15 for use with endoscopes is guided to the container 91 in the same way as in the first embodiment. The sample acquired with the instrument 15 is introduced into the sample container 91.

The container 91 now containing the sample moves back into the magazine 93. In the magazine 93, it may be moved to another position as the drive means 95 rotates the cylinder 94. Alternatively, it may be ejected from the magazine 93 through the ejection opening 101.

The container 91 may be moved back in interlock with the pulling of the instrument 15 from the container 91. The container 91 to be used next to obtain a sample is moved to the lowest position in the magazine 93 as the cylinder 94 rotates.

The biopsy-sample obtaining unit 92 is made of transparent material (glass, resin, or the like), either as a whole or in part. Alternatively, the unit 92 is made of non-transparent material and may have an opening. Thus, the user can determine how many containers 91 are held in the biopsy-sample obtaining unit 92 and which container 91 contains a sample, merely by looking at the unit 92 from outside.

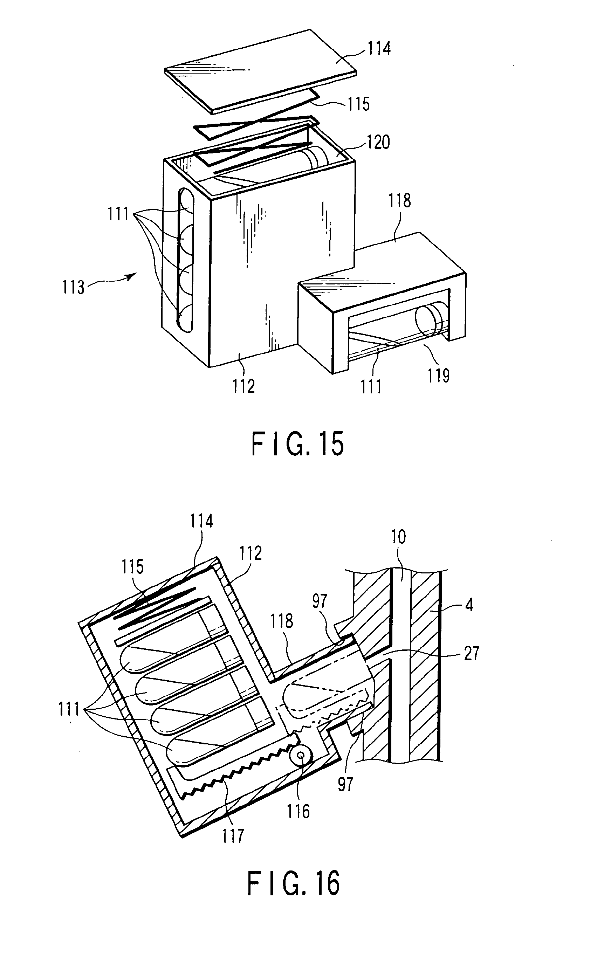

FIGS. 15 and 16 show a sixth embodiment of this invention. The present embodiment differs from the fifth embodiment (see FIGS. 14A and 14B) in that a plurality of sample containers 111 are arranged in a row in a magazine 112 as shown in FIG. 15, not in a hollow cylindrical, biopsy-sample obtaining unit 92 as in the fifth embodiment. In any other respect, the present embodiment is identical in configuration to the fifth embodiment. The component identical to those of the fifth embodiment are designated by the same reference numbers and will not be described.

In this embodiment, the containers 111 are arranged in the magazine 112, one upon another, forming a column. Each container 111 is biased downwards by its own weight or by an elastic member 115 secured to the cover 114 of a biopsy-sample obtaining unit 113.

The biopsy-sample obtaining unit 113 is removably set in the recess 97 of the operation section 4 as in the fifth embodiment. FIG. 16 shows the biopsy-sample obtaining unit 113 attached to the operation section 4 of the endoscope 2. The lowest container 111 in the biopsy-sample obtaining unit 113 is moved to an endoscope-coupling unit 118 and thereby connected to the endoscope 2 as a gear 116 drives a lever 117. In this state, the instrument 15 for use with endoscopes is inserted into the sample container 111 in the same way as in the first embodiment. The sample obtained with the instrument 15 is therefore introduced into the sample container 111.

The container 111, which now contains the sample, is removed from a lower-container ejecting unit 119. The cover 114 of the biopsy-sample obtaining unit 113 may be opened. Then, new containers 111 can be inserted into the magazine 112 through the upper opening 120 made in the biopsy-sample obtaining unit 113.

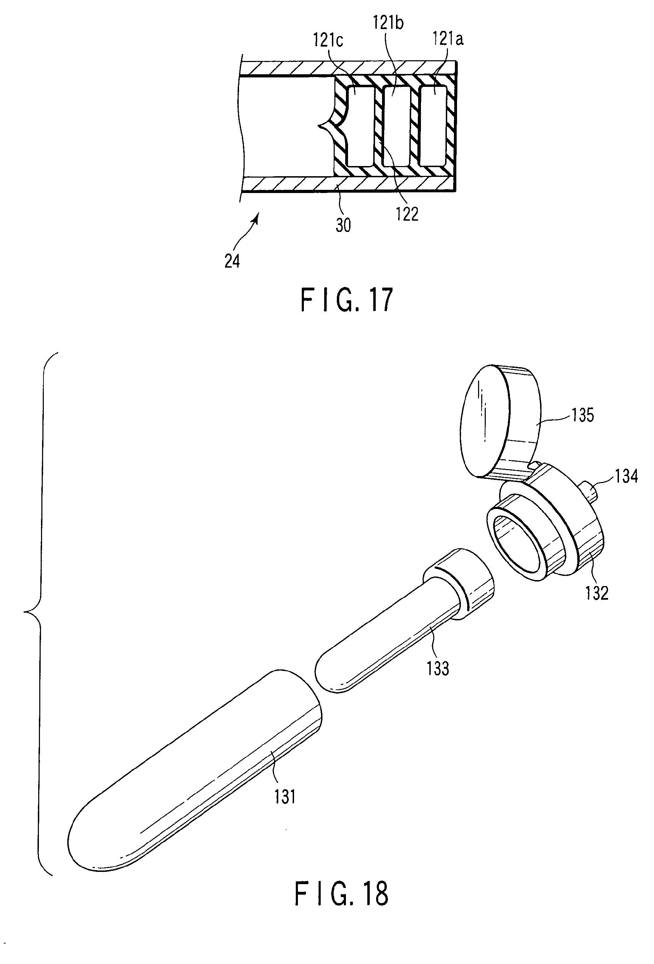

FIG. 17 shows a seventh embodiment of the present invention. This embodiment differs from the endoscope system 1 according to the second embodiment (see FIGS. 8 and 9) in that a modified closing means is used for the sample container 24.

As shown in FIG. 17, a plurality of airtight chambers made of elastic material, or three chambers 121a, 121b and 121c in the present embodiment, are provided in the open end of the sample container 24. The pressure in each airtight chamber is higher than that in the immediately outer airtight chamber. This prevents air from flowing into the container 24 from outside when a sample is inserted into the container 24.

The three airtight chambers 121a, 121b and 121c contain a substance that reacts with air (consisting mainly oxygen and nitrogen). This enables the user to determine whether the interior of the container 24 is maintained at normal pressure, before the container 23 is used.

The airtight chambers 121a, 121b and 121c have a part each, which can be broken when it is pierced with a puncture needle 18. In addition, the airtight chambers 121a, 121b and 121c contain at least two substances, respectively. These substances change in color when mixed together. Hence, the user can determine whether a sample has been introduced into the container 24. Further, the puncture needle 18 may be released from the operation wire of the instrument 15 and therefore left in the container 24 after the sample has been introduced into the container 14. This enables the user to determine whether a sample has been introduced into the container 24.

FIG. 18 shows an eighth embodiment of the present invention. This embodiment differs from the endoscope system 1 according to the first embodiment (see FIGS. 1A to 7C) in that the sample container 24 is changed in configuration as will be described below.

That is, the container used in this embodiment has a case 131 and an adapter 132. The case 131 is a bottomed hollow cylinder that has the same size as the sample container 24 for the first embodiment. The adapter 132 is attached to the open end of the case 131. The case 131 incorporates a small container 133 that is smaller than the sample container 24 used in the first embodiment. The adapter 132 seals the case 131 and is attached to the sample-insertion part of the small container 133.

The adapter 132 has a cylindrical part 134 and a cover 135. The cylindrical part 134 may be inserted into the channel-branch hole 27 of the instrument channel 10 of the endoscope 2. The cover 135 can seal the case 131. When the cylindrical part 134 of the adapter 132 is inserted into the channel-branch hole 27 of the instrument channel 10 of the endoscope 2, the small container 133 in the case 131 is attached to the operation section 4 of the endoscope 2. In this state, the instrument 15 for use with endoscopes is inserted into the small container 133. A sample can be thereby introduced into the small container 133 provided in the adapter 132. Hence, the small container 133 can be used as a sampling container such as a vacuum blood-sampling tube that has been hitherto used. Moreover, the cover 135 can close the small container 133, rendering the same airtight.

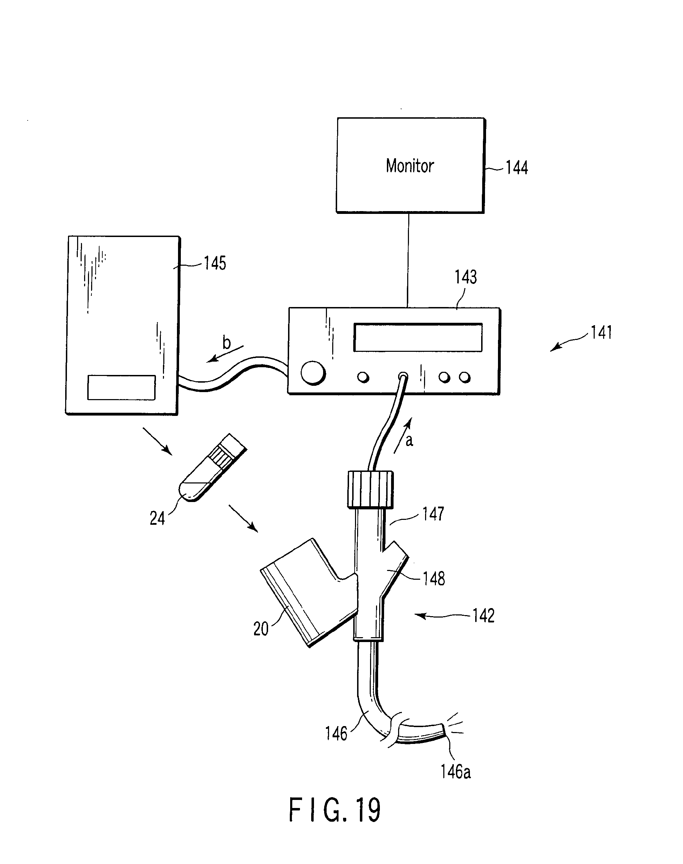

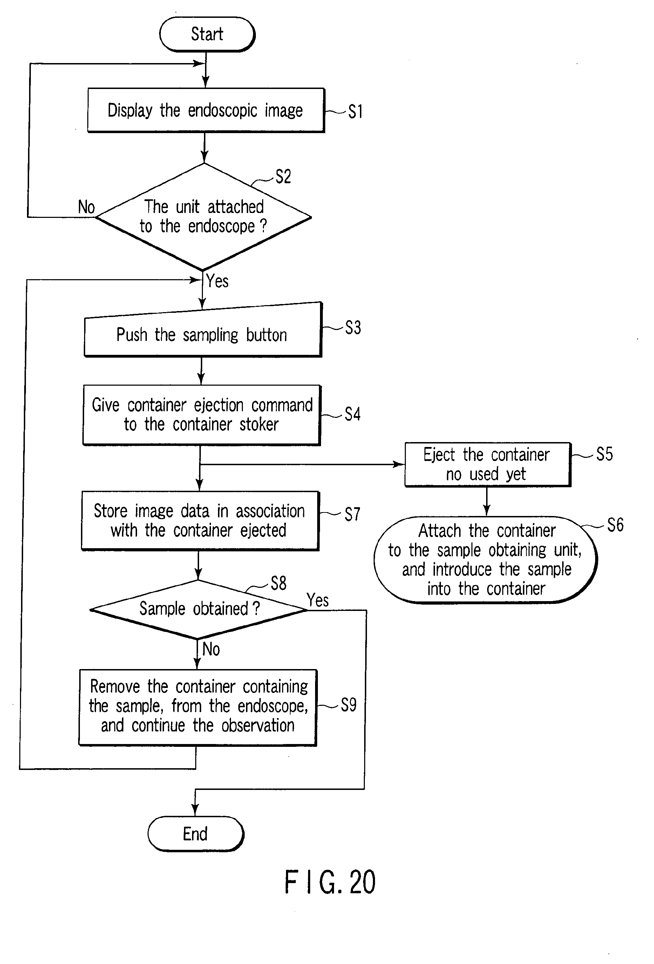

FIGS. 19 and 20 show a ninth embodiment of the present invention. This embodiment differs from the endoscope system 1 according to the first embodiment in respect of the overall configuration as will be described below. FIG. 19 is a schematic representation of the entire endoscope system 141 according to this embodiment.

The endoscope system 141 according to this embodiment has an electronic endoscope 142, a video-system center 143, a monitor 144, and a container stocker 145. The electronic endoscope 142 has an elongated insertion section 146 and an operation section 147. The insertion section 146 may be inserted into the patient. The operation section 147 is coupled to the proximal end of the insertion section 146. The distal end 146a of the insertion section 146 contains an observation optical system that incorporates an imaging element such as a CCD. The imaging element, which is provided in the distal end 146a of the insertion section 146, photographs the object of observation which exists in the patient. The imaging element converts the image of the object, photographed, into an electric signal. The signal is supplied to the video-system center 143. The video-system center 143 treatment the electric signal, whereby the monitor 144 displays the image of the object.

The operation section 147 of the electronic endoscope 142 has an instrument insertion port 148 and a sample obtaining unit 20. The unit 20 has the same configuration as in the first embodiment (see FIGS. 1A to 7C).

How this embodiment configured as described above operates will be explained with reference to the flowchart of FIG. 20. To obtain a biopsy sample with the instrument 15, the electronic endoscope 142 photographs the target biopsy tissue and the monitor 144 displays the image of the tissue photographed (Step S1).

Next, in Step S2, the video-system center 143 determines whether the biopsy-sample obtaining unit 20 has been attached to the operation section 147 of the electronic endoscope 142. If it is not determined in Step S2 that the biopsy-sample obtaining unit 20 has been attached to the operation section 147 of the electronic endoscope 142, the operation returns to Step S1. If it is determined in Step S2 that the biopsy-sample obtaining unit 20 has been attached to the operation section 147 of the electronic endoscope 142, the operation goes to the next step, i.e., Step S3.

In Step S3, the user depresses a sampling button when he or she recognizes, in the image displayed on the monitor 144, the site at which to obtain a biopsy sample. The sampling button may be provided on the video-system center 143.

The signal a generated when this button is depressed is supplied to the video-system center 143. The video-system center 143 receives the signal a and gives the container stocker 145 a container ejection command b (Step S4)

The container stocker 145 receives the container ejection command b and ejects the container 24 that has yet to be used (Step S5).

In the present embodiment, the containers 24 have an identification means each, such as a barcode or a microchip. The stocker 145 has a mechanism for reading the identification means and must eject a container 24 in accordance with the command given by the video-system center 143. The user attaches the container 24 ejected and not used yet, to the sample obtaining unit 20 of the endoscope 2. The sample obtained is introduced into the container 24 (Step S6).

The video-system center 143 stores the image represented by the electric signal received, in association with the container 24 to be ejected (Step S7). Thus, the data about the container 24 into which the sample has been introduced can therefore be stored in association with the container 24. This facilitates the recognition of the sample later.

Thereafter, in Step S8, it is determined whether the biopsy sample has been obtained. If it is not determined in Step S8 that the biopsy sample has been obtained, the operation goes to Step S9. In Step S9, the user removes the container 24 containing the sample, from the operation section 147 of the electronic endoscope 142. The user continues the observation through the electronic endoscope 142. The operation then returns to Step S3.

If it is determined in Step S8 that the biopsy sample has been obtained, the procedure of obtaining a biopsy sample is terminated.

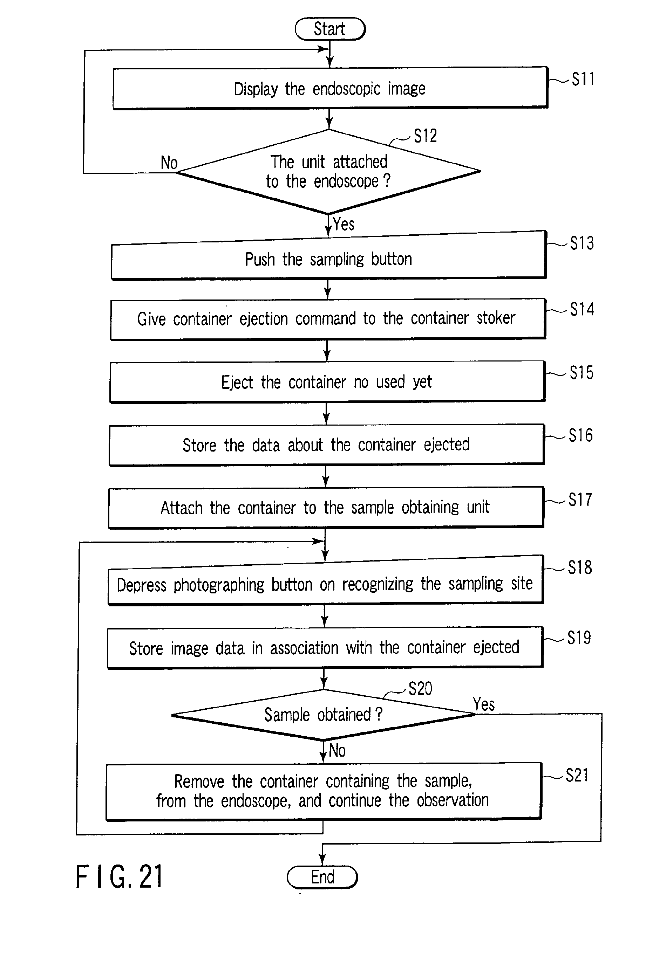

FIG. 21 shows a tenth embodiment of the present invention. This embodiment differs from the endoscope system 141 according to the ninth embodiment (see FIGS. 19 and 20) in that the image of the sampling site is associated with the container 24 in a different manner.

How the image of the sampling site is associated with the container 24 in the endoscope system 141 according to this embodiment will be explained with reference to the flowchart of FIG. 21. Note that Steps S11 to S12 performed in this embodiment are identical to Steps S1 to S2 performed in the ninth embodiment.

In Step S12, it may be determined that the biopsy-sample obtaining unit 20 has been attached to the operation section 147 of the electronic endoscope 142. Then, the operation goes to Step S13. In Step S13, the user depresses the container ejection button provided on the video-system center 143 or container stocker 145.

When the user depresses the container ejection button, a signal a is generated and supplied to the video-system center 143. The video-system center 143 receives this signal a and gives the container stocker 145 a container ejection command b (Step S14).

The container stocker 145 receives the container ejection command b and ejects the container 24 that has yet to be used (Step S15). Since the containers 24 have a barcode or an IC tag each. The containers 24 in the container stocker 145 can be identified independently of one another. The data about the container 24 ejected is transferred from the container stocker 145 to the video-system center 143 and is stored therein (Step S16).

The user then attaches the container 24, which has not been used yet, to the sample obtaining unit 20 of the endoscope 2 (Step S17). In the next step, i.e., Step S18, the user depresses the photographing button when he or she recognizes the site where to obtain a biopsy sample, on the display screen of the monitor 144.

The signal generated when this button is depressed is supplied to the video-system center 143. The video-system center 143 receives this signal and stores the image photographed, in association with the container 24 ejected (Step S19).

Thereafter, in Step S20, it is determined whether the biopsy tissue has been sampled or not. If it is not determined in Step S20 that the biopsy tissue has been sampled, the operation returns to Step S18. If it is determined in Step S20 that the biopsy tissue has been sampled, the treat of obtaining the biopsy tissue is terminated.

The biopsy-sample obtaining unit 20 or the endoscope 2 may have a mechanism that can read identification means, and the information identified may be transferred to the video-system center 143, thereby to associate the container 24 with the information identified.

Thus, the patient's name, the sampled part, the sampling date, and the like can be correlated if the container 24 containing the biopsy sample has identification means such as a barcode or an IC tag. For example, the sampling site may be identified on the image obtained by the endoscope 2. Then, the sampling site can be correlated with the container that contains the sample.

This is helpful in examining a treated region, e.g., cancer region, to determine how the region changes with time. This is particularly desirable when several samples are obtained at the same site, as in the case of prostate carcinoma.

Various data items can be recorded in the identification means. These data items includes the patient's name, the pathology number, the sampled part, the sampling date, the number of samples, the examination results (date, diagnosis, examiner's name). The data items can be recorded in the form of an electronic medical record.

If the identification means is used as described above, a sample examined will not be taken for another.

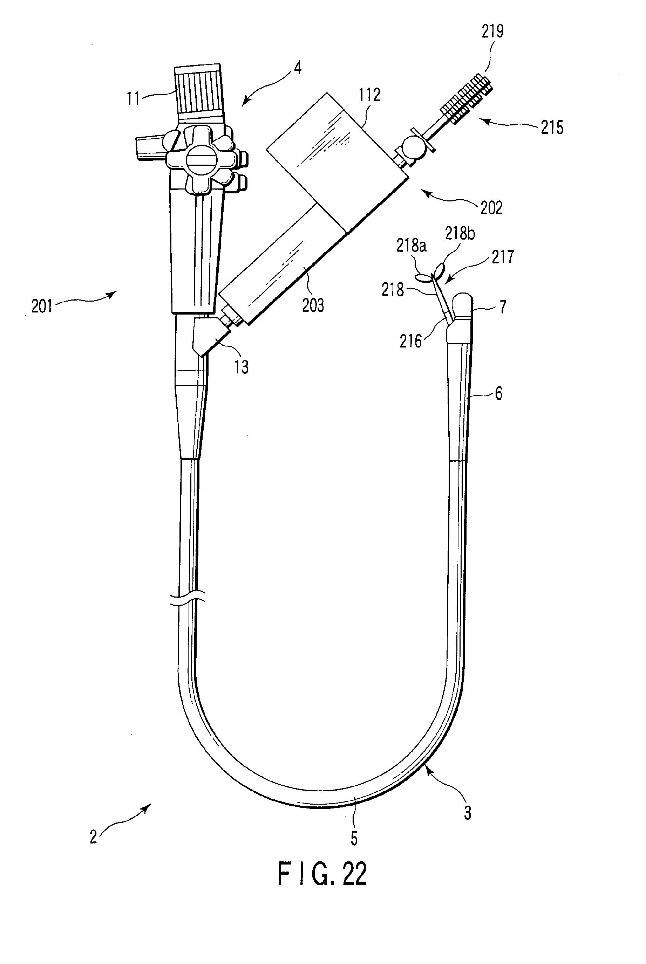

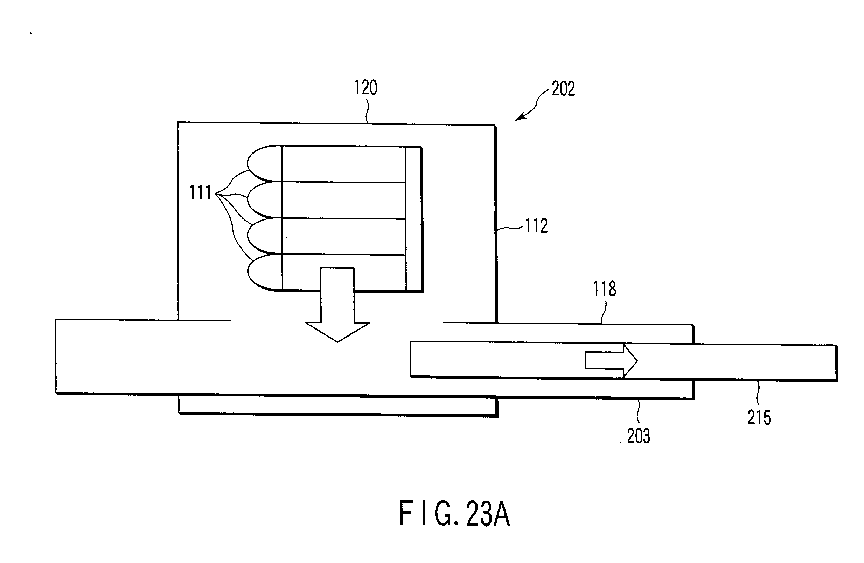

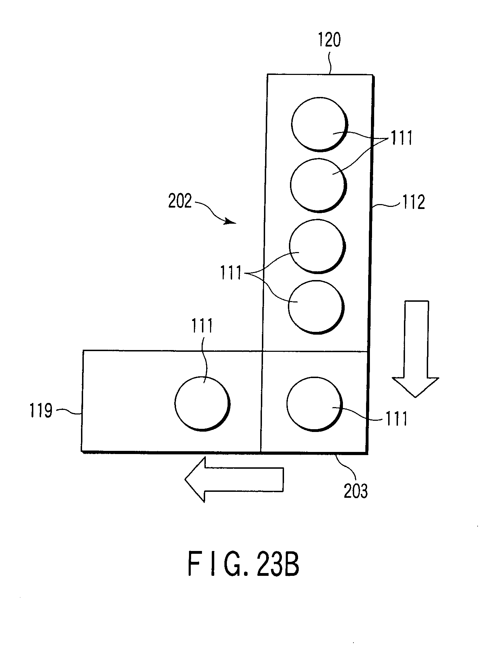

FIG. 22 and FIGS. 23A and 23B show a tenth embodiment of the present invention. FIG. 22 schematically shows the overall configuration of an endoscope system 201 according to this embodiment. The present embodiment is essentially identical in configuration to the endoscope system 1 (FIG. 1) according to the first embodiment. Therefore, the components identical to those of the first embodiment are designated by the same reference numbers and will not be described.

The present embodiment differs from the endoscope system 1 according to the first embodiment in that the biopsy-sample obtaining unit 202 attached to the operation section 4 of the endoscope 2 is arranged at the instrument-insertion port 13.

The biopsy-sample obtaining unit 202 of this embodiment is essentially identical in structure to the biopsy-sample obtaining unit 113 of the sixth embodiment (see FIGS. 15 and 16) in which the containers are arranged in a row. As FIG. 23B shows, the biopsy-sample obtaining unit 202 has two windows. Through one window (i.e., upper opening 120), containers 111, each holding a biopsy sample in it, are inserted into the unit 202. Through the other window (i.e., lower container-ejecting opening 119). At least one container 111 holding a biopsy sample in it is provided in the biopsy-sample obtaining unit 202. In this embodiment, a plurality of containers 111 are provided in the unit 202. Each container 111 is inserted through the upper opening and ejected through the lower opening.

A communication passage 203 is provided at the lower part of the magazine 112 of the biopsy-sample obtaining unit 202. An instrument 215 for use with endoscopes can extend through the communication passage 203. Any container 111 in the magazine 112 can be inserted into, and removed from, this communication passage 203.

The instrument 215 for use with endoscopes, according to this embodiment, has a biopsy forceps 218. The forceps 218 has a pair of grasping forceps 218a and 218b, which can assume an opened position and a closed position. The biopsy forceps 218 is provided at the distal end of an instrument insertion part 216 that is an elongated flexible tube. In other words, a pair of grasping forceps 218a and 218b are arranged at the distal end of the instrument insertion section 216. An instrument-operating unit 219 coupled to the proximal end of the instrument insertion section 216 can move the grasping forceps 218a and 218b to the opened position and the closed position.

How this embodiment configured as described above operates will be explained. The instrument 215 passes through the communication passage 203 of the biopsy-sample obtaining unit 202 and is inserted into the instrument channel 10 through the instrument-insertion port 13. The grasping forceps 218a and 218b are opened and closed, holding a biopsy sample between them. The sample is thereby obtained. After the sample has been thus obtained, the instrument 215 is pulled into the communication passage 203 of the biopsy-sample obtaining unit 202. At this time, the instrument 215 is never pulled out of the communication passage 203 of the biopsy-sample obtaining unit 202 as the instrument 215 that has obtained the sample is moved toward the proximal end (inlet port) of the communication passage 203 of the biopsy-sample obtaining unit 202.

As the instrument 215 moves toward the proximal end of the communication passage 203 of the biopsy-sample obtaining unit 202, an empty container 111 is inserted into the communication passage 203. The empty container 111 is set at a position where the biopsy sample can be transferred from the instrument 215 into the empty container 111. In this state, the instrument 215 is moved forward into the empty container 111. Further, the grasping forceps 218a and 218b are set in the opened position. The biopsy sample is thereby released and falls into the container 111.

Thereafter, the instrument 215 is moved toward the proximal end of the communication passage 203 of the biopsy-sample obtaining unit 202. In this state, the container 111 now containing the biopsy sample is moved toward the lower container-ejecting part 119. The container 111 containing the biopsy sample is moved outside from the lower container-ejecting part 119. A plurality of samples may be obtained from one patient. In this case, the instrument 215 is inserted from the instrument-insertion port 13, passing through the communication passage 203 of the biopsy-sample obtaining unit 202. The sequence of these operations can be repeated.

The configuration described above is advantageous in the following respect. The instrument 15 is never pulled out of the communication passage 203 of the biopsy-sample obtaining unit 202 as the instrument 215 that has obtained the sample is moved toward the communication passage 203 of the biopsy-sample obtaining unit 202. The biopsy sample obtained with the grasping forceps 218a and 218b of the instrument 215 can be introduced into the sample container 111 held in the biopsy-sample obtaining unit 202, without pulling the instrument 215 out of the communication passage 203 of the biopsy-sample obtaining unit 202. As a result, the instrument 215 is inserted into the sample container 111, sealed from the atmosphere, as in the endoscope system 1 according to the first embodiment. The sample obtained with the grasping forceps 218a and 218b of the instrument 215 is ejected into the container 111. This prevents the sample from contaminating the environment and from being contaminated. The biopsy sample obtained by the instrument 215 for use with endoscopes can therefore be fast stabilized and preserved fresh. Thus, the sample can undergo accurate examinations and diagnoses.

Moreover, the biopsy sample is never contaminated, because the sample, such as a biopsy tissue or a body fluid, can be obtained without being exposed to the environment. The deterioration of the biopsy sample can be reduced, too. Samples can be obtained from different subjects under the same condition. This helps to standardize the samples among them.

An endoscope system according to an embodiment of this invention, which incorporates a rigid endoscope, will be described below. The insertion section of the rigid endoscope, which may be inserted into the patient, has a sheath that is a rigid tube such a metal tube. The rigid endoscope is inserted into the patient, applying a surgical invasion to the patient, in order to perform a surgical operation or an observation in the patient.

Most rigid endoscopes that are designed to be inserted into peritoneal cavities, are system each comprises various components. The rigid endoscope system is composed of an observation section (a rigid endoscope in a narrow sense), a surgical instrument, and a hollow cylindrical member (trocar). The observation section has a main light source and an optical focusing means. The surgical instrument is manipulated to treat organs or perform operations on the organs. The hollow cylindrical member is used as a guide for inserting the observation section and the surgical instrument into the patient.

Usually, a plurality of trocars are inserted into a cavity in the patient from outside, and the observation section (i.e., rigid endoscope) is inserted into the cavity through one of the trocars and the instrument is inserted into the cavity through another trocar. The instrument may be one designed to cut or suture the organs, a suction device for drawing liquids, a pressurizing device for pressurizing peritoneal cavities.

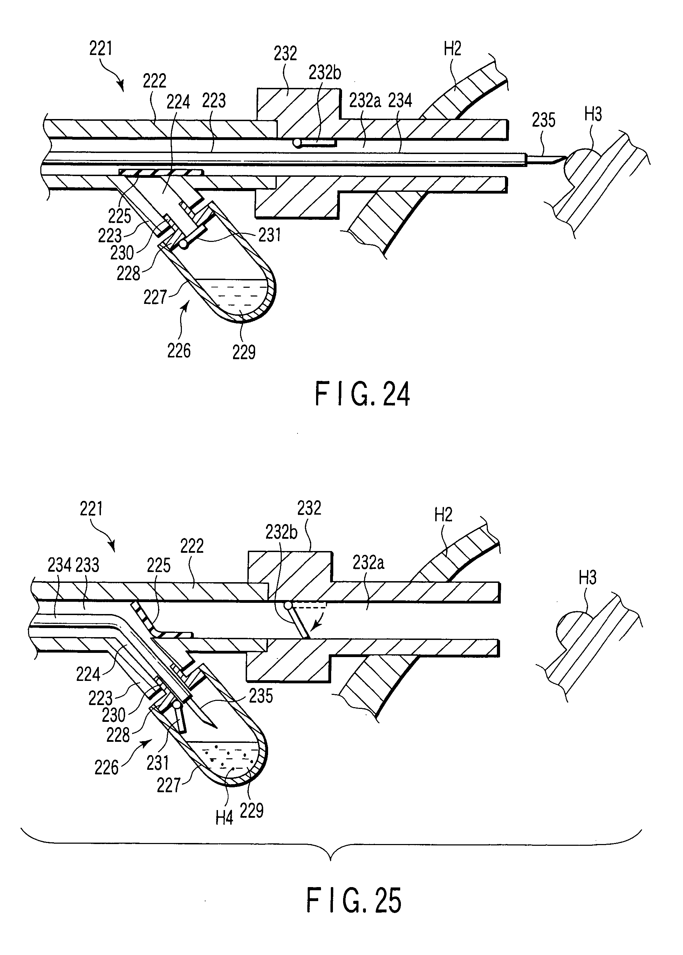

FIGS. 24 and 25 shows an eleventh embodiment of the present invention. This embodiment uses a tissue-sampling unit 221 that may be inserted into the patient, in addition to a rigid endoscope. As shown in FIG. 24, the tissue-sampling unit 221 has a tubular unit body 222. A container-coupling part 223 protrudes from the outer circumferential surface of the unit body 222. In the container-coupling part 223, a communication passage 224 is made, which communicates with the interior of the unit body 222.

A passage-switching plate 225 is provided in the unit body 222. The plate 225 can open and close the communication passage 224 made in the container-coupling part 223, thereby to switch passages. That is, the passage-switching plate 225 may open the inner passage of the unit body 222 and close the communication passage 224 of the container-coupling part 223, as is illustrated FIG. 24. Alternatively, it may close the inner passage of the unit body 222 and open the communication passage 224 of the container-coupling part 223, as is illustrated FIG. 25. A sample container 226 is removably coupled to the container-coupling part 223.

The sample container 226 has a container body 227 and a cover 228. The container body 227 is a bottomed hollow cylinder. The cover 228 closes the open end of the sample container 226. The container body 227 contains tissue-preserving liquid 229. The tissue-preserving liquid 229 may be replaced by a reagent that can treat samples obtained from the patient. The reagent may be, for example, one that stabilizes nucleic acid. Alternatively, the reagent may be one that treatment nucleic acid, protein, cells, tissues or blood. Further, the reagent may be contained in gel.

A communication cylindrical part 230 is provided in the center part of the cover 228. The communication cylindrical part 230 is removably coupled to the container-coupling part 223 of the tissue-sampling unit 221.

A valve 231 is mounted on the inner surface of the cover 228. The valve 231 can open and close the interior of the communication cylindrical part 230. The valve 231 is biased in a direction, usually closing the interior of the communication cylindrical part 230. Thus, the valve 231 prevents the tissue-preserving liquid 229 from flowing back into the patient while the sample container 226 remains coupled to the tissue-sampling unit 221, and prevents air from flowing into the container 226 when the container 226 is decoupled from the tissue-sampling unit 221.

As shown in FIG. 24, a trocar 232 is set in the patient's abdominal wall H2 so that the tissue-sampling unit 221 of this embodiment may be used. A valve 232b is fitted in the trocar 232 and can open and close the passage 232a of the trocar 232. This valve 232b is biased in a direction, usually closing the passage 232a of the trocar 232. The unit body 222 of the tissue-sampling unit 221 is connected, at distal end, to the trocar 232. The sample container 226 is attached to the container-coupling part 223 of the tissue-sampling unit 221.

In this state, an instrument 223 is inserted into the unit body 222 of the tissue-sampling unit 221. At this point, the passage-switching plate 225 in the unit body 222 is held, closing the communication passage 224 of the container-coupling part 223.

The instrument 233 has an elongated insertion section 234 that is made of elastic material and therefore has flexibility. The insertion section 234 has a puncture needle 235 at its distal end. The insertion section 234 of the instrument 233 passes through the unit body 222 of the tissue-sampling unit 221 and the interior of the trocar 232 and is inserted into the patient. The insertion section 234 of the instrument 233 opens the valve 232b of the trocar 232, and the passage 232a of the trocar 232 is thereby opened. The puncture needle 235 provided at the distal end of the insertion section 234 is thrust into the biopsy tissue H3 existing in the peritoneal cavity, which lies in the view field of the rigid endoscope (not shown). A sample H4 is obtained from the biopsy tissue H3 existing in the peritoneal cavity.

After the biopsy sample H4 has been obtained, the insertion section 234 of the instrument 233 is moved back toward the proximal end of the endoscope until the puncture needle 235 reaches the proximal edge of the passage-switching plate 225. In this state, the valve 232b of the trocar 232 returns to the closed position. The passage 232a of the trocar 232 is thereby closed.

Thereafter, as shown in FIG. 25, the passage-switching plate 225 closes the inner passage of the unit body 222 and opens the communication passage 224 of the container-coupling part 223. The passage is thereby switched. In this state, the insertion section 234 of the instrument 233 is pushed forward in the inner passage of the unit body 222. At this point, the insertion section 234 of the instrument 233 is guided into the sample container 226 from the communication passage 224 of the container-coupling part 223. The insertion section 234 of the instrument 233 is inserted into the container body 227 from the communication cylindrical part 230 of the cover 228 for the sample container 226. At this time, the puncture needle 235 on the insertion section 234 pushes open the valve 231 as shown in FIG. 25. The puncture needle 235 on the insertion section 234 is therefore inserted into the container body 227.

Next, the sample H4 is introduced into the container body 227. In this method, the sample container 226 can be replaced by another so that a new sample can be obtained.

The trocar 232 and the tissue-sampling unit 221 remain shielded from the atmosphere while the biopsy sample H4 is being obtained by using the tissue-sampling unit 221. Hence, no air flows into the patient, and no pressure drop occurs in the patient. The switching of the passage for the puncture needle used in this embodiment can be the endoscope channel mechanism described above.

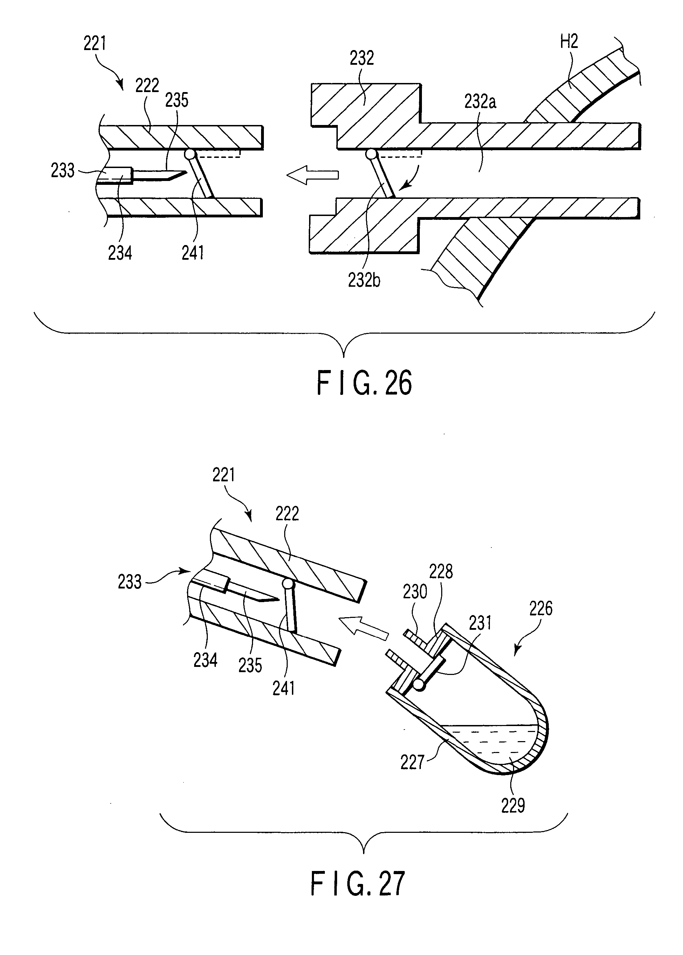

FIGS. 26 to 28 show a twelfth embodiment of this invention. The present embodiment differs from the eleventh embodiment (see FIGS. 24 and 25) in that the tissue-sampling unit 221,is modified as will be described below.

In the present embodiment, a valve 241 that can open and close the unit body 222 is provided in the distal end of the unit body 222 of the tissue-sampling unit 221. This valve 241 is biased in a direction to close the interior of the unit body 222. In any other respect, this embodiment is identical in configuration to the endoscope system 1 (FIG. 1) according to the first embodiment. Therefore, the components identical to those of the first embodiment are designated by the same reference numbers and will not be described.

During the use of the tissue-sampling unit 221 according to this embodiment, the trocar 232 is set in the patient's abdominal wall as illustrated in FIG. 26. In this state, the unit body 222 of the tissue-sampling unit 221 is connected, at distal end, to the trocar 232.

Then, the insertion section 234 of the instrument 233 is inserted into the patient through the trocar 232 from the unit body 222 of the tissue-sampling unit 221, in the same manner as in the eleventh embodiment. As the insertion section 234 of the instrument 233 is so inserted, the valve 241 in the unit body 222 and the valve 232b of the trocar 232 are pushed open. The interior of the unit body 222 and the passage 232a of the trocar 232 are thereby opened. The puncture needle 235 provided at the distal end of the insertion section 234 is thrust into the biopsy tissue H3 existing in the peritoneal cavity, which lies in the view field of the rigid endoscope (not shown). A sample H4 is obtained from the biopsy tissue H3 existing in the peritoneal cavity.

After the biopsy sample H4 has been obtained, the insertion section 234 of the instrument 233 is moved back toward the proximal end of the endoscope. The puncture needle 235 is therefore pulled back until the puncture needle 235 is reaches the proximal side of the valve 241 provided in the unit body 222. In this state, the valve 232b of the trocar 232 has returned to the closed position, whereby the passage 232a of the trocar 232 is closed. Similarly, the valve 241 in the unit body 222 returns to the closed position, whereby the interior of the unit body 222 is closed.

In this state, the tissue-sampling unit 221 is removed from the trocar 232 as shown in FIG. 26. At this point, the valves 232 and 241 seal the trocar 232 and the unit 221 airtight, respectively.

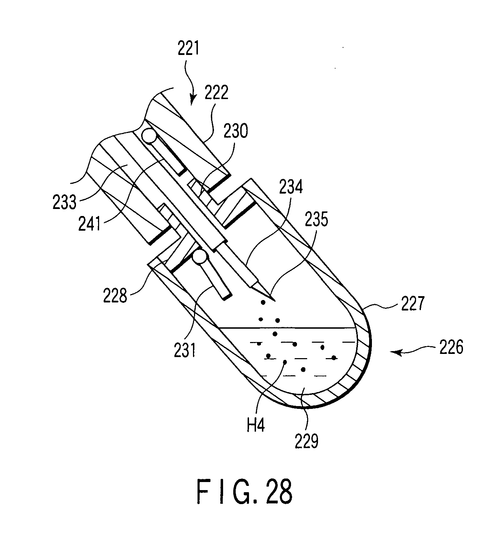

Thereafter, the sample container 226 is attached to the distal end of the unit body 222 of the tissue-sampling unit 221 as shown in FIG. 27. Then, as shown in FIG. 28, the biopsy sample H4 is ejected from the puncture needle 235 inserted in the container 226.

In the present embodiment, the sample container 226 never hinders the manipulation of obtaining the sample H4, i.e., a part of the biopsy tissue H3, from the patient's peritoneal cavity. Note that the puncture needle 235 can be replaced by one that has no flexibility.

In the instances mentioned above, not only the puncture needle 235, but also forceps or any other instruments can be used to collect biopsy tissues.

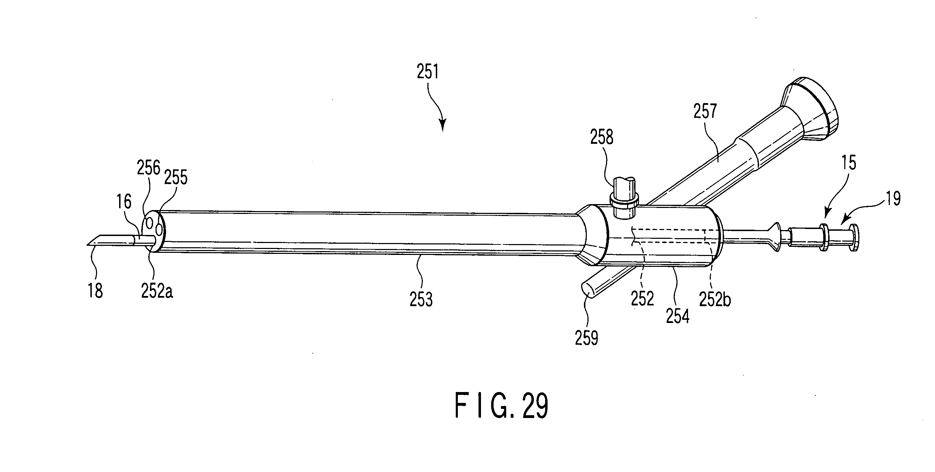

FIG. 29 shows a thirteenth embodiment of the present invention. This embodiment differs from the first embodiment (see FIGS. 1A to 7C) in that the instrument 15 for use with endoscopes is inserted into the patient through the instrument channel 252 of the rigid endoscope 251 shown in FIG. 29. The components identical to those of the first embodiment are designated by the same reference numbers and will not be described.

The rigid endoscope 251 according to this embodiment has an insertion section 253, which is, for example, a metal pipe and is therefore a straight elongated rigid one. An operation section 254 having a large diameter is secured to the proximal end of the insertion section 253. In the distal end part of the insertion section 253 there are provided an observation window 255 of the observation optical system, an illumination window 256 of the illumination system, and the distal-end port 252a of the instrument channel 252.

An ocular unit 257 and a light-guide connection cap 258 protrude from the outer circumference of the operation section 254. Between the ocular unit 257 and the observation window 255, an image-transmitting optical system (not shown) extends to transmit an image formed on the observation window 255. Further, between the light-guide connection cap 258 and the illumination window 256, a light guide (not shown) extends to guide illumination light.

At the distal end of the operation section 254, the distal-end port 252a of the instrument channel 252 is located. The instrument-insertion part 16 of an instrument 15 for use with endoscopes is inserted into the instrument channel 252 through the distal-end port 252a. The instrument 15 is ultimately inserted into the patient through the distal-end port 252a.

A container connection unit 259 protrudes from the outer circumferential surface of the operation section 254. A sample container 24 can be coupled to the container connection unit 259. The container connection unit 259 has a container connection port, with which the container 24 may be coupled.

How the present embodiment operates will be explained. To use the instrument 15 for use with endoscopes, the rigid endoscope 251 is inserted into the patient. A trocar, for example, is used to insert the instrument 15 into the patient. The trocar has a tube and a puncture needle that can be inserted into the tube. The trocar is set in the patient's abdominal wall, with the puncture needle inserted in the tube.

After the trocar has been set in the patient's abdominal wall, the puncture needle is pulled from the tube. The rigid endoscope 251 is then inserted into the tube set in the patient's abdominal wall. The rigid endoscope 251 is inserted into the patient, while guided through the tube of the trocar.

Subsequently, the instrument 15 for use with endoscopes is inserted into the patient through the instrument channel 252 of the rigid endoscope 251. Then, a biopsy sample is taken into the sample container 24 connected to the container connection unit 259 of the rigid endoscope 251 in the same manner as in the first embodiment. Thereafter, the instrument 15 is pulled from the sample container 24 held in the biopsy-sample obtaining unit 20. At this point, the instrument 15 for use with endoscopes is pulled outside through the distal-end port 252a of the instrument channel 252.

Then, the instrument 15 for use with endoscopes is inserted into the patient through the instrument channel 252 of the rigid endoscope 251. The puncture needle 18 at the distal end of the instrument channel 252 of the rigid endoscope 251 is thrust into the biopsy tissue. The biopsy tissue is drawn by using a syringe. Then, the instrument 15 for use with endoscopes is pulled out. The biopsy tissue thus sampled is introduced into the sample container 24. The sample can undergo various treatment.

In the present embodiment, the instrument 15 for use with endoscopes should be flexible. Nevertheless, the instrument 15 need not be flexible if such a magazine as shown in FIGS. 23A and 23B is connected to the proximal part of the operation section 254.

The present invention is not limited to the embodiments described above. Various changes and modifications can, of course, be made, without departing from the scope and spirit of the present invention.

### Industrial Applicability

The present invention is useful in the field of endoscope systems that can hold a biopsy sample obtained with an instrument inserted into the channel of an endoscope and in the field of methods of obtaining biopsy samples.

## Claims

1. An endoscope system **characterized by** comprising:
an endoscope having:
an insertion section which is to be inserted into a subject;
an operation section which is coupled to a proximal end of the insertion section and arranged outside the subject;
an instrument channel which is arranged in the insertion section and extends from the operation section to a distal end of the insertion section; and
observation means for observing an interior of the subject; and
an instrument having:
an instrument insertion unit which is to be inserted into the subject through the instrument channel;
sample-obtaining means arranged at the distal end of the instrument insertion unit and configured to obtain a biopsy sample; and
receptacle means arranged at the operation section and configured to contain the biopsy sample obtained by the instrument.

2. The endoscope system according to claim 1, **characterized in that** the operation section has passage-switching means provided in middle part of the instrument channel, for switching a communication state between a first communication state in which an input port of the instrument channel communicates with an output port of the instrument channel and a second communication state in which the inlet port of the instrument channel communicate with the receptacle means.

3. The endoscope system according to claim 2, **characterized in that** the receptacle means has at least one container configured to contain a biopsy sample and is able to be removed from the operation section and to be replaced by another.

4. The endoscope system according to any one of claims 1 to 3, **characterized in that** the receptacle means has a filter configured to separate a biopsy sample into a solid sample and a liquid sample.

5. The endoscope system according to claim 1, **characterized in that** the receptacle means comprises identification means for identifying a biopsy sample to be introduced into the receptacle means.

6. The endoscope system according to claim 1, **characterized in that** the receptacle means comprises a receptacle device configured to hold a biopsy sample and having:
a container which is configured to contain a biopsy sample; and
a receptacle unit which is removably attached to the endoscope and configured to hold the container.

7. The endoscope system according to claim 1, **characterized in that** the receptacle means has a receptacle device which is configured to hold the biopsy sample obtained and which contains a reagent for treatment the biopsy sample.

8. The endoscope system according to claim 7, **characterized in that** the reagent is an agent for treatment biopsy samples.

9. The endoscope system according to claim 7, **characterized in that** the reagent is an agent for treatment nucleic acid, protein, cells, tissues or blood.

10. The endoscope system according to any one of claims 7 to 9, **characterized in that** the reagent is contained in gel.

11. The endoscope system according to any one of claims 7 to 10, **characterized in that** the receptacle means comprises stirring means for stirring the reagent and the biopsy sample in the container.

12. The endoscope system according to claim 1, **characterized in that** the receptacle means comprises temperature-adjusting means.

13. The endoscope system according to claim 1, **characterized in that** the biopsy sample obtained by the sample-obtaining means is a biopsy tissue, cells, body fluid, blood or secretion.

14. A biopsy-sample container for use in an endoscope system **characterized by** comprising:
an endoscope having:
an insertion section which is to be inserted into a subject;
an operation section which is coupled to a proximal end of the insertion section and arranged outside the subject;
an instrument channel which is arranged in the insertion section and extends from the operation section to a distal end of the insertion section; and
observation means for observing an interior of the subject; and
an instrument having:
an instrument insertion unit which is to be inserted into the subject through the instrument channel; and
sample-obtaining means arranged at the distal end of the instrument insertion unit and configured to obtain a biopsy sample; and
receptacle means arranged at the operation section and configured to contain the biopsy sample obtained by the instrument; and
said container having:
a container body; and
a coupling part which is configured to be attached and detached to and from the endoscope system.

15. The biopsy-sample container according to claim 14, **characterized in that** the container body comprises identification means for identifying the biopsy sample.

16. The biopsy-sample container according to claim 14, **characterized in that** the container body has a filter configured to separate a biopsy sample into a solid sample and a liquid sample.

17. The biopsy-sample container according to claim 14, **characterized in that** the container body is depressurized.

18. The biopsy-sample container according to claim 14, **characterized in that** the container body contains a reagent for treatment the biopsy sample.

19. The biopsy-sample container according to claim 18, **characterized in that** the reagent is an agent for stabilizing nucleic acid.

20. The biopsy-sample container according to claim 18, **characterized in that** the reagent is an agent for treatment nucleic acid, protein, cells, tissues or blood.

21. The biopsy-sample container according to claim 18, **characterized in that** the reagent is contained in gel.

22. A method of obtaining a biopsy sample, **characterized by** comprising:
a insertion step of inserting an instrument insertion unit of an instrument for use with endoscopes, into a subject through an instrument channel of an endoscope configured for observation of the interior of the subject;
a biopsy-sample obtaining step of obtaining a biopsy sample by using sample-obtaining means arranged at a distal end part of the instrument insertion unit; and
a sample introducing step of introducing the biopsy sample obtained by the sample-obtaining means arranged at the distal end part of the instrument insertion unit, into receptacle means attached to the distal end of the instrument insertion unit.

23. The method of obtaining a biopsy sample, according to claim 22, **characterized by** comprising a sample-treatment step of treatment the biopsy sample by introducing the sample into the receptacle means which contains a reagent.

24. The method of obtaining a biopsy sample, according to claim 23, **characterized by** further comprising a stirring step of stirring the biopsy sample and the reagent in the receptacle means.

25. The method of obtaining a biopsy sample, according to claim 22, **characterized by** further comprising a culturing step of culturing cells contained in the biopsy sample introduced into the receptacle means.

26. A method of treatment a biopsy sample, **characterized by** comprising:
a insertion step of inserting an instrument insertion unit of an instrument for use with endoscopes, into a subject through an instrument channel of an endoscope configured for observation of the interior of the subject;
a biopsy-sample obtaining step of obtaining a biopsy sample by using sample-obtaining means arranged at a distal end part of the instrument insertion unit; and
a sample introducing step of introducing the biopsy sample obtained by the sample-obtaining means, into receptacle means attached to the endoscope; and
a treatment step of performing, on the biopsy sample contained in the receptacle means, at least one treat selected from the group consisting of reagent treatment, refrigeration, freezing, freeze-drying, thermal insulation and culture.
